# EUROPEAN PATENT SPECIFICATION

(11) **EP 3 996 617 B1**
(45) Date of publication and mention of the grant of the patent: **03.09.2025**
(21) Application number: 20836222.8
(22) Date of filing: 09.07.2020
(51) Int. Cl.: A61B 18/06, A61B 18/04, A61B 18/08, A61N 1/40, A61B 18/00, A61B 18/28, A61B 18/14

(54) **APPARATUS FOR TREATMENT OF TUMORS USING A TREATMENT OBJECT AND POWERING OF THE TREATMENT OBJECT**
VORRICHTUNG ZUR BEHANDLUNG VON TUMOREN MIT EINEM BEHANDLUNGSOBJEKT UND SPEISUNG DES BEHANDLUNGSOBJEKTS
APPAREIL POUR LE TRAITEMENT DE TUMEURS À L'AIDE D'UN OBJET DE TRAITEMENT ET ALIMENTATION EN ÉNERGIE DE L'OBJET DE TRAITEMENT

(30) Priority: 10.07.2019 IL 26798919; 26.12.2019 IL 27173819
(43) Date of publication of application: 18.05.2022
(73) Proprietor: Cohen, David, 4630902 Herzliya (IL)
(72) Inventor: Cohen, David, 4630902 Herzliya (IL)
(74) Representative: Plasseraud IP
(86) International application number: PCT/IL2020/050776
(87) International publication number: WO 2021/005610

(56) References cited:
- WO-A1-2019/120489
- US-A- 4 494 539
- US-A1- 2010 100 092
- US-A1- 2011 251 516
- US-A1- 2012 071 873
- US-A1- 2012 226 270
- US-A1- 2013 006 228
- US-A1- 2019 099 618
- US-B2- 8 073 551
- US-B2- 9 675 817
- SELF ET AL.: "Warming Intravenous Fluids for improved Patient Comfort in the Emergency Department: A Pilot Crossover Randomized Controlled Trial", WESTERN JOURNAL OF EMERGENCY MEDICINE, vol. 14, no. 5, September 2013 (2013-09-01), XP055788757
- ZHANG ET AL.: "Temperature-dependent cell death patterns induced by functionalized gold nanoparticle photothermal therapy in melanoma cells", SCIENTIFIC REPORTS, vol. 8, 7 June 2018 (2018-06-07), pages 8720, XP055788761
- HUMAN BODY TEMPERATURE, 6 March 2018 (2018-03-06), XP055788768, Retrieved from the Internet <URL:https://en.wikipedia.org/w/index.php?title=Human_body_temperature&oldid=829123941>
- ANONYMOUS: "Pumps / compressors P&ID symbols", ENGGCYCLOPEDIA.COM, pages 1 - 5, XP055891620, Retrieved from the Internet <URL:https://web.archive.org/web/20160307123617/https://www.enggcyclopedia.com/2011/02/pumps-compressors-pid-symbols> [retrieved on 20220215]

## Description

### RELATED APPLICATION/S

This application claims the benefit of priority under 35 USC §119(e) of Israel Patent Application No. 267989 filed July 10, 2019, and of Israel Patent Application No. 271738, filed 26 December 2019.

### FIELD AND BACKGROUND OF THE INVENTION

The present invention relates to systems for introducing a treatment object into a tumor, more particularly, but not exclusively, to systems for heating the treatment object including remote treatment.

Treatment of cancer in the human or animal body centers around the killing and removal of tumor and tumorous cells. Once identified as malignant, the next stage of treatment is the removal of the tumor. As long as the tumor is accessible and reasonably large and the cancer has not spread, surgery is an option. However, numerous small tumors or tumors that are difficult to access, cannot be treated with surgery, so that other, less invasive options, such as chemotherapy and radiotherapy may be used. Radiotherapy may be aimed precisely at the tumor from outside and takes advantage of the fact that the rapidly dividing tumor cells are more sensitive to the radiation than healthy cells. Chemotherapy also takes advantage of the sensitivity of the rapidly dividing tumor cells but is much harder to direct precisely at the tumor, hence having considerable side effects. Both of radiotherapy and chemotherapy are highly effective with the right kinds of cancers and the earlier the stage of the cancer the better. However some kinds of cancer are resistant to these therapies or it may be too late to apply them. In some cases chemotherapy fails because of the side-effects of the chemotherapy agents on healthy tissue requiring the dose to be limited, and surgery may be difficult if there are large numbers of small tumors or the tumors are hard to access.

Cancer research is thus constantly on the look-out both for means of delivering chemotherapy drugs more precisely to the tumor, thus safely allowing for an increase in the dose. Likewise cancer research is looking for ways of dealing with tumor that has spread to multiple sites around the body.

US Patent Application No. 2012/0071873A1 to Russel Blake Thomson et al, of March 22, 2012, teaches a system for treatment of a hollow anatomical structure. A catheter inserts a therapeutic element into a hollow element of the body requiring treatment.

US Patent Application No. 2019/099618A1 to Paul Stauffer et al of April 4, 2019 discloses a tumor bed implant for multimodal treatment of at risk tissue surrounding a resection cavity.

US Patent Application No. 2010/100092 A1 to Paul F Turner et al of April 22, 2010 discloses an apparatus and method for heating a deposit in fatty tissue in the body.

### SUMMARY OF THE INVENTION

The present invention relates to attacking the tumor using heat and/or directly delivered chemotherapy drugs. The present invention relates to a device wherein a treatment object into the tumor is insertable in the body and heat the body and/or release the drug. Rapidly dividing cells such as tumor cells are generally more sensitive to heat and chemotherapy drugs than healthy cells and hence there is a temperature range in which the tumor cells can be effectively attacked with minimal collateral damage to healthy cells. Such a temperature range is between 39 and 50 degrees, advantageously between 42 and 45 degrees, more advantageously between 43 and 44 degrees, and a particularly recommended temperature is 43.5 degrees. The present invention relates to systems for introducing such a treatment object into a patient, and to systems for heating the treatment object, including heating by wire, by laser through an optical fiber, using piped fluid, using a battery or using induction.

The present invention provides a device for treatment of a tumor according to claim 1, comprising:
a treatment object configured for placement about the tumor, the treatment object comprising a heating surface configured to reach a treatment temperature, the treatment temperature being higher than a patient body temperature;
an energy source configured to provide energy to reach said treatment temperature, said energy source being located externally to said treatment object; and
an outwardly expandable spring around said treatment object, wherein the spring is configured to expand under temperature control;
characterized in that the device is configured to move the treatment object 401 in steps through a medium using magnetic pulses, thereby to locate the treatment object 401 at a desired location in the medium to treat said tumor.

The term external may refer to being connected to the treatment object via a wire or pipe or both, or may refer to a wireless connection, so that the treatment object may be powered externally of the patient.

The dependent claims define embodiments of the present invention.

The scope of the present invention is defined by the appended claims.

Methods of treatment described hereinafter are not part of the present invention but are presented for better understanding the present invention.

Embodiments disclosed hereinafter are of exemplary nature and presented for better understanding the present invention.

Unless otherwise defined, all technical and/or scientific terms used herein have the same meaning as commonly understood by one of ordinary skill in the art to which the invention pertains.

As will be appreciated by one skilled in the art, the protocols and timing operations for the present disclosure may be embodied as a system, method or computer program product. Accordingly, some examples of the present disclosure may include control systems which take the form of an entirely hardware embodiment, an entirely software embodiment (including firmware, resident software, micro-code, etc.) or an example combining software and hardware aspects that may all generally be referred to herein as a "circuit," "module" or "system." Furthermore, some examples of the present disclosure may take the form of a computer program product embodied in one or more computer readable medium(s) having computer readable program code embodied thereon. Implementation of the method and/or system can involve performing and/or completing selected tasks manually, automatically, or a combination thereof. Moreover, according to actual instrumentation and equipment of the disclosed method and/or system, several selected tasks could be implemented by hardware, by software or by firmware and/or by a combination thereof, e.g., using an operating system.

For example, hardware for performing selected tasks could be implemented as a chip or a circuit. As software, selected tasks could be implemented as a plurality of software instructions being executed by a computer using any suitable operating system. In an exemplary embodiment one or more tasks according to the method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some examples of the present disclosure. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some examples of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some examples of the present disclosure may be described below with reference to flowchart illustrations and/or block diagrams of methods, apparatus (systems) and computer program products according to examples of the present disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as controlling heating of a remote body, might be expected to use completely different methods, e.g., making use of expert knowledge and/or the pattern recognition capabilities of the human brain, which would be vastly more efficient than manually going through the steps of the methods described herein.

### BRIEF DESCRIPTION OF THE SEVERAL VIEWS OF THE DRAWING(S)

Some examples and embodiments of the present disclosure are herein described, by way of example only, with reference to the accompanying drawings. With specific reference now to the drawings in detail, it is stressed that the particulars shown are by way of example and for purposes of illustrative discussion of examples and embodiments of the present disclosure. In this regard, the description taken with the drawings makes apparent to those skilled in the art how examples of the present disclosure may be practiced.

In the drawings:
Fig. 1 is a simplified flow diagram showing a first method of treating tumor using a device according to the present embodiments;
Fig. 2 is a simplified flow diagram showing a second method of treating tumor using a device according to the present embodiments;
Fig. 3 is a simplified flow diagram showing a third method of treating tumor using a device according to the present embodiments
Fig. 4 is a simplified diagram of a treatment object according to an example embodiment;
Fig. 5A is a simplified diagram showing an optional example embodiment of an interior of the treatment object of Fig. 1;
Fig. 5B is a simplified diagram showing an interior of a treatment object according to an example embodiment;
Fig. 5C is a simplified diagram showing an interior of a treatment object according to an example embodiment;
Fig. 5D is a simplified diagram showing a magnetic layer with holes covered by a shell according to an example embodiment;
Fig. 5E is a simplified diagram showing a variation in which two separate internal containers are available according to an example embodiment;
Fig. 5F is a variation of the body of Fig. 1;
Fig. 6 is a simplified diagram showing a treatment object being moved around by pulses according to an example embodiment;
Fig. 7A is a simplified diagram showing an example embodiment including a container part and a magnetized body;
Fig. 7B is a simplified illustration of a variation of the embodiment of Fig.7A;
Fig. 7C is a cross sectional view of the embodiment of Fig.7A;
Figs. 8, 9A and 9B are examples of magnetic coils that may be used according to some example embodiments;
Figs. 10A-E are simplified illustrations of an example embodiment of an injector and various example embodiments of treatment bodies;
Figs. 11A-D, 12 and 13A-F are simplified illustrations of a treatment object attached via wires according to example embodiments;
Fig. 14 is a simplified illustration of a treatment object incorporating an antenna;
Fig. 15 is a simplified diagram illustrating an external antenna for providing energy for energizing the device of the present embodiments;
Figs. 16A-D are simplified illustrations of a treatment object incorporating an antenna according to examples of the present disclosure;
Figs. 17A-F and 18A-C are simplified illustrations of various ways in which a cover may be added to a treatment object according to example embodiments; and
Figs. 19A-D are simplified illustrations of a treatment object including a wire antenna according to example embodiments.
Fig. 20 is a simplified diagram showing a treatment object according to the present embodiments with triple wire connector;
Fig. 21 is a simplified diagram showing a tumor in the walls of a body lumen;
Figs. 22A - 22E show successive stages of introducing a treatment object according to the present embodiments to the tumor of Fig. 21;
Fig. 23 is a simplified diagram showing an elongated variation of the treatment object of Fig. 22A for use with multiple tumors in the same lumen;
Fig. 24 is a simplified diagram showing a spring structure that forms part of the treatment object of Figs 21A to 23;
Fig 25 is a simplified diagram illustrating a treatment object comprising a spring structure held within an envelope;
Fig. 26 is a simplified diagram illustrating a treatment object with multiple treatment windows in an envelope according to examples of the present disclosure;
Figs. 27A and 27B are two diagrams showing a treatment object according to the present embodiments in situ in a tumor and powered wirelessly from an external power source;
Fig. 28 is a simplified diagram showing a spring structure with openings for drip release of therapy agents;
Figs. 29A and 29B are two simplified diagrams showing valve members for governing drip feed at the openings of Fig. 28;
Fig. 30 is a simplified diagram showing the spring structure of FIG. 28 with the drip feed valve members of FIG. 29 inserted therein;
Fig. 31 is a simplified diagram illustrating the component parts of the internal antenna of a device according to the present embodiments;
Fig. 32 is a simplified diagram showing a cross-section of a treatment object according to the present embodiments, wherein the bladder has a built-in heating element;
Fig. 33 is a simplified diagram showing a treatment object having an insulation envelope, the envelope having windows at intervals specifically inserted for the patient;
Fig. 34 is a simplified diagram showing a person inside a coil that induces heating in multiple treatment objects according to the present embodiments;
Fig. 35 is a simplified diagram showing a circuit for a transmitter to provide suitable RF fields to power heating in the treatment objects according to the present embodiments;
Fig. 36 is a simplified diagram illustrating an antenna and a treatment object that consists of a bladder, according to examples of the present disclosure;
Fig. 37 is a variation of the treatment body of FIG 36, in which flaps are provided on the treatment object to allow for surgical sewing to fix the object in position; and
Figs. 38 - 40 illustrate a variation of the treatment object of Fig. 36 in which an outer layer of piping is provided for drip feeding of treatment fluids such as chemotherapy agents.

### DESCRIPTION OF SPECIFIC EMBODIMENTS OF THE PRESENT DISCLOSURE

### Introduction

The term "treatment object" is used herein to refer to a device which is intended to be used inside a patient's body.

The term "patient" is used herein to refer to an entity which the treatment device is used to treat, by way of examples: human, animal and plant.

The term "magnetic" is used herein to refer to materials which can be attracted or repulsed or otherwise mechanically affected by magnetic fields. Such materials include, by way of some non-limiting examples, ferromagnetic materials, such as, by way of a non-limiting example, iron; additional materials which react to a magnetic field, such as paramagnetic materials; and composite materials including some component(s) which can be affected by a magnetic field, optionally together with some components which are not affected by a magnetic field.

### Overview

As explained in the background, treatment of cancer in the human or animal body centers around the killing and removal of tumor and tumorous cells. Once identified as malignant, the next stage of treatment is the removal of the tumor. As long as the tumor is accessible and reasonably large and the cancer has not spread, surgery is an option. However, numerous small tumors or tumors that are difficult to access, cannot be treated with surgery, so that other, less invasive options, such as chemotherapy and radiotherapy may be used. Radiotherapy may be aimed precisely at the tumor from outside and takes advantage of the fact that the rapidly dividing tumor cells are more sensitive to the radiation than healthy cells. Chemotherapy also takes advantage of the sensitivity of the rapidly dividing tumor cells but is much harder to direct precisely at the tumor, hence having considerable side effects. Cancer cells are also differentially sensitive to heat, and the present disclosure relates to attacking the tumor using heat, or using directly inserted chemotherapy agents. Embodiments may insert a treatment object into the tumor and heat the body. Rapidly dividing cells such as tumor cells are generally more sensitive to heat than healthy cells and hence there is a temperature range in which the tumor cells can be effectively attacked with minimal collateral damage to healthy cells. Such a temperature range is between 39 and 50 degrees, advantageously between 42 and 45 degrees, more advantageously between 43 and 44 degrees, and a particularly recommended temperature is 43.5 degrees. The embodiments relate further to methods and systems for introducing such a treatment object into a patient, and to methods and systems for heating the treatment object, including heating by wire, by laser through an optical fiber, using piped fluid, using a battery or using induction. Embodiments relate to controlling the heating of the treatment object

### System for guiding

As will be discussed in greater detail below, the body may be inserted into the tumor directly using a syringe and needle or other placement device. The tumor may have been detected in a body scan and in embodiments, markers may have been inserted. In embodiments the markers inserted into the tumors during the scan are in fact bodies that can be heated.

### System for heating

The present embodiments may allow for the body to be heated in a number of ways, including heating by wire, by laser through an optical fiber, using piped fluid, using a battery or using induction or radio frequency. In one embodiment, the patient sits within a coil that generates a large electromagnetic field that can heat many small heating bodies each inserted into a different tumor of a cancer that has already begun to spread. Embodiments relate to controlling the heating of the treatment object, as will be discussed in greater detail below.

### Treatment object features

The body may be constructed of any material with a suitable heat capacity to allow it to be heated reasonably easily and quickly and which may allow for dissipation of the heat into the surroundings. The material may differ depending on the mode of supplying heat, thus metal or metal powder in a carrier may be needed for inductive heating, but any conductive material may be useful in the case of using a piped fluid. Metal may include iron, a combination of two or more metals as different portions of the treatment object or alloyed together to obtain particular properties. The device may use a binding agent of polymer and metal particles, and in an embodiment, a part of the device may be heatable by radio and another part may not-necessarily heatable by radio. Embodiments may be designed by constructing alloys with a specific thermal capacity by mixing two or more materials with different thermal capacities.

### Method for controlling the heating

Any kind of heating rests on a balance between heat provided and heat dissipated. The heat provided may be estimated based on tables associating one or more of antenna parameters, treatment object parameters, tumor parameters, body-part parameters, heating pulse duration, and non-heating duration. A device may be custom made for a particular tumor, based on say the size, location and type of tumor treatment object.

The present section relates to guidance of a treatment object and, more particularly, but not exclusively, to a treatment object that may be guided towards a treatment site and then activated.

Some embodiments may provide a treatment object that is reactive to magnetic or other guidance, and guide the treatment object to a treatment site, where it may optionally be activated to apply treatment. The treatment object may for example be heated, by way of a non-limiting example by using magnetic waves, in order to destroy a tumor or gallstone or the like, or cause a blood clot to form, for example to stop a hemorrhage, or to destroy a blood clot. In some embodiments the treatment object may include radioactive material. In some embodiments the treatment object may be a container containing treatment material. In the case of a container, when the target is reached, the treatment object may optionally be caused by applied energy to open and release material at the treatment target. The treatment object may alternatively destroy the target by heating, say caused externally by induction. In an embodiment, the treatment object may enter the target at successive locations and apply heating at each location. The treatment object may travel or rotate around the outside of a tumor and separate the tumor from its blood supply, potentially inducing tumor necrosis.

The treatment object may be moved in pulses. The size and weight of the treatment object are known and it is possible to set the size of a magnetic pulse so as to define a given step size. The treatment object may be moved at different rates through different media and movement may be monitored using X-ray or CT scanning or even MRI, so that the treatment object can be delivered precisely at a target. In some embodiments the treatment object may contain a radioactive source which can be located and/or traced, for example using triangulation. A further optional method of location and/or tracing includes using ultrasound to locate and/or trace the treatment object. In some embodiments the treatment object may be moved and activated using electro-magnetic radiation of any suitable kind. A magnetic field may provide propulsion and/or guide the treatment object. In some embodiments, activation may use the same magnetic field to induce currents in the object and thus heat the treatment object.

In some embodiments, heating may be achieved using infra-red, radio-frequency or even X-ray radiation and all references herein to induction and heat activation are to be construed accordingly. The radiation wavelengths may be selected in accordance with the size of the treatment object and the material that the treatment object is made of. The material may be an alloy that has specific properties in terms of heat capacity and/or heat absorption from radiated energy.

When the target area is in bone, say in the case of bone tumors, the treatment object may be pulsed to carry out repeatable knocks on the bone and thereby to make a precise hole.

According to an aspect of some examples of the present disclosure there is provided a treatment object including or consisting of magnetic material, and a magnetic field source, the magnetic field source configured to provide pulses to move the treatment object in steps through a viscous medium, thereby to locate the treatment object at a desired location in the viscous medium. As an alternative to pulses, continuous movement may be used.

In some embodiments, the magnetic field source is further configured to induce heating in the treatment object. In some embodiments, microwave or radio waves may be used.

In some embodiments, the treatment object contains a therapeutic substance.

In some embodiments, the treatment object includes a shell of material, the shell being dissolvable or melt-able, optionally melt-able by heating, to release the therapeutic substance.

In some embodiments, the shell is optionally dissolvable and/or melt-able by receipt of a signal from the magnetic field source, potentially causing release of material such as medication to take place at a desired location.

In embodiments, the shell of the treatment object is dissolvable and/or melt-able by induction from the magnetic field source causing heating, thereby to ensure that the release takes place at the desired location.

Some embodiments of the treatment object optionally include a magnetized core, a hollow volume containing a therapeutic substance and an outer shell.

Some embodiments of the treatment object may include a central hollow space for a therapeutic substance, a magnetized shell and a dissolvable shell.

Some embodiments of the treatment object may include a central hollow, the hollow being openable, when the of the treatment object reaches a desired location, and the magnetic field source is configured to provide a pulse to move the opened hollow through the medium so that the hollow in the of the treatment object potentially collects a sample of the medium at the target location.

According to an aspect of the present disclosure there is provided an exemplary method of targeting a location in a human or animal for treatment including:
Inserting a treatment object into the human or animal;
Using a magnetic field to pulse the treatment object in incremental steps towards the target;
Using x-ray based imaging to trace progress of the treatment object through the body; and
When the treatment object is at the location then activating the treatment object to apply a treatment.

In this way, in some embodiments, the treatment object is able to deliver substances to locations that cannot be approached using a needle, or that are too small to be approached by a needle. There are certain tumors that can be very clear under scanning, say under MRI or PET⁻/CT scanning, but are too small for the surgeon to see when operating. Some of the embodiments provide a way for such tumors to be approached.

Activating may include heating the treatment object and/or or releasing a therapeutic substance contained in the treatment object.

In some embodiments, the method may involve repeatedly approaching the target from different sides and applying treatment at some or all of the approaches. In some embodiments, using 3D imaging, a program of attacking the tumor from different sides may be provided and followed.

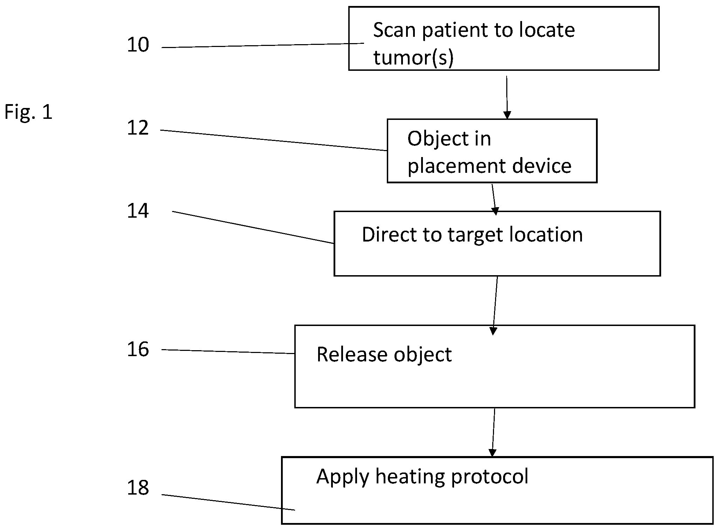

Reference is now made to Fig. 1, which is a simplified flow diagram that illustrates a process for using treatment bodies according to the present embodiments. Initially, a patient is scanned 10 to locate tumors. A treatment object according to any one of the embodiments discussed herein, is placed 12 in a placement device that allows medical personnel to make a precise location within the body. Depending on the embodiment, the object may be directly injected into the tumor or may be placed in a lumen and then fed to the tumor, or may be placed in tissue and fed to the tumor - 14. At the target location, which be on or in contact with the tumor, the object is released 16 and a treatment protocol is applied 18. The treatment protocol may be a heating protocol, but may alternatively or additionally be a treatment protocol such as a chemotherapy protocol, involving the treatment object releasing drugs. The present embodiments may thus provide a way of delivering chemotherapy directly to the tumor.

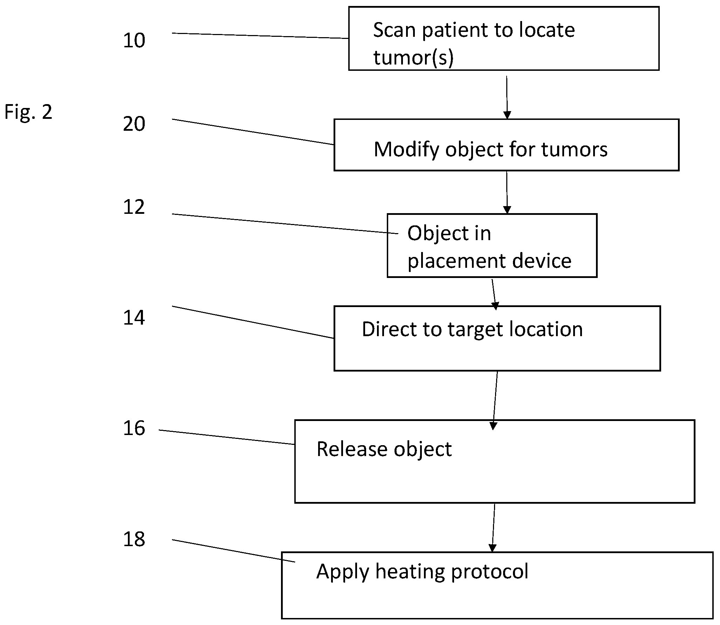

Reference is now made to Fig. 2, which is a simplified flow diagram that illustrates a second process for using treatment bodies according to the present embodiments. Initially, a patient is scanned 10 to locate tumors. Then a treatment object according to any one of the embodiments discussed herein, is constructed or modified 30 to suit the anatomy of a single tumor or the distribution of multiple tumors, so that the individual patient obtains a customized treatment object. The object is then placed 12 in a placement device that allows medical personnel to make a precise location within the body. Depending on the embodiment, the object may be directly injected into the tumor or may be placed in a lumen and then fed to the tumor, or may be placed in tissue and fed to the tumor - 14. At the target location, which be on or in contact with the tumor, the object is released 16 and a treatment protocol is applied 18. The treatment protocol may be a heating protocol, but may alternatively or additionally be a treatment protocol such as a chemotherapy protocol, involving the treatment object releasing drugs.

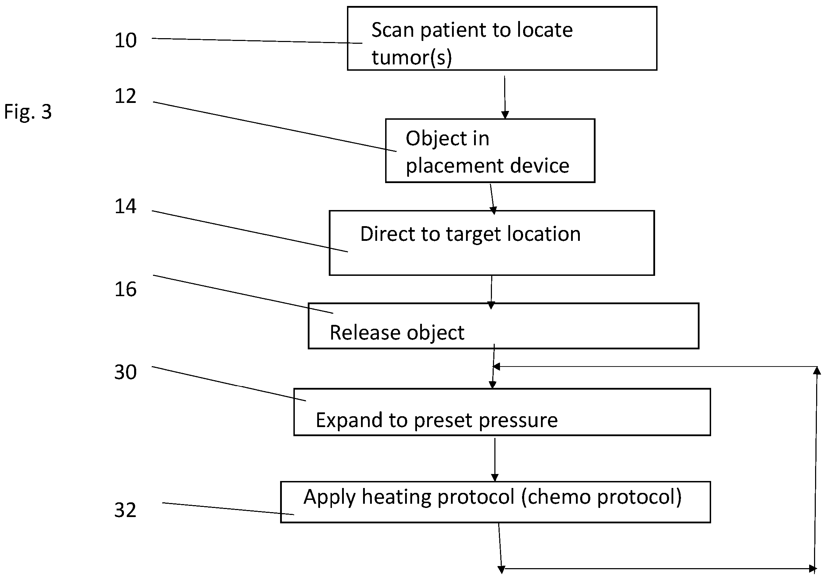

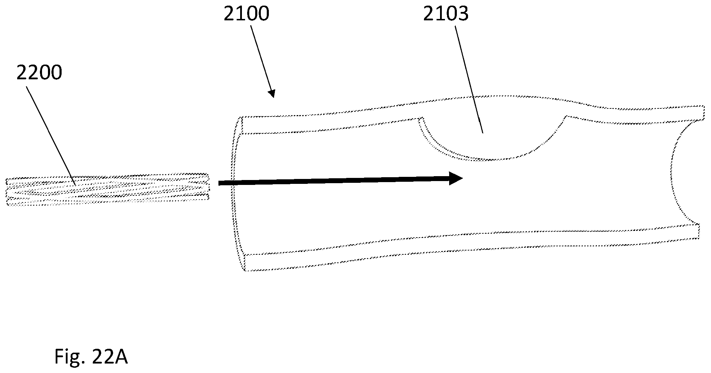

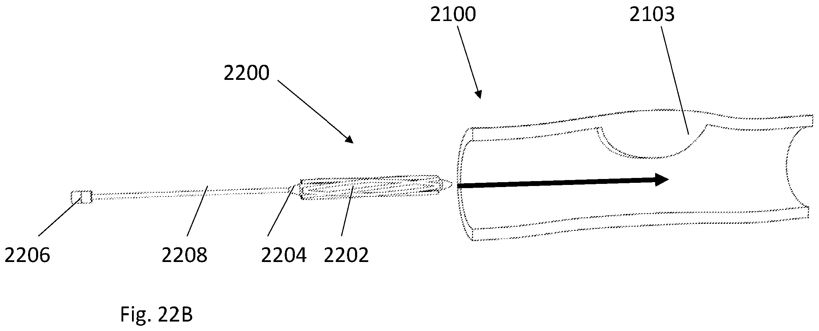

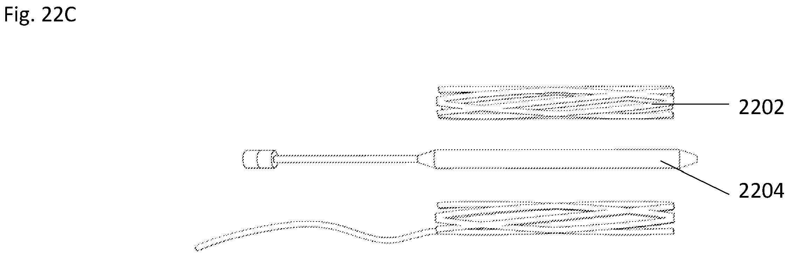

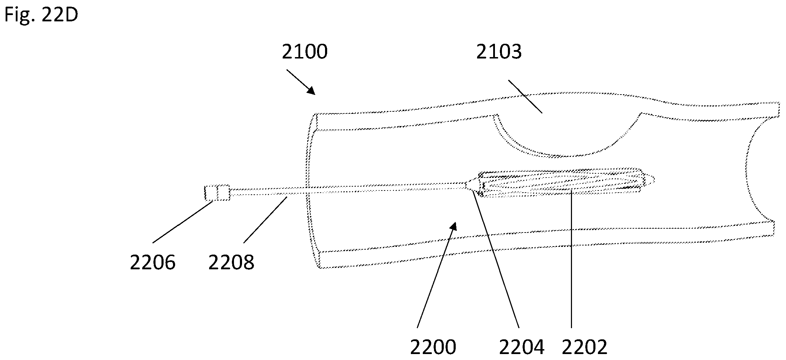

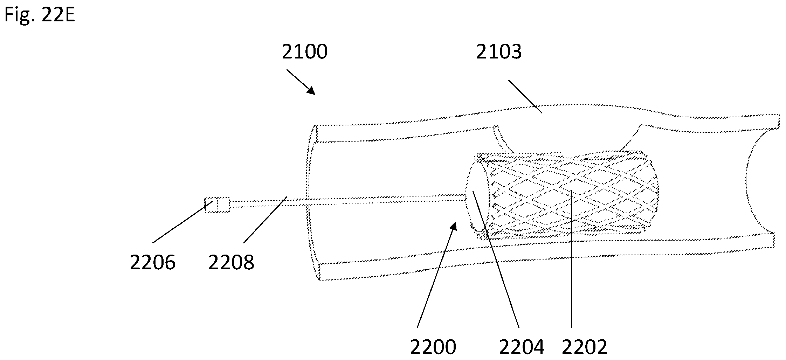

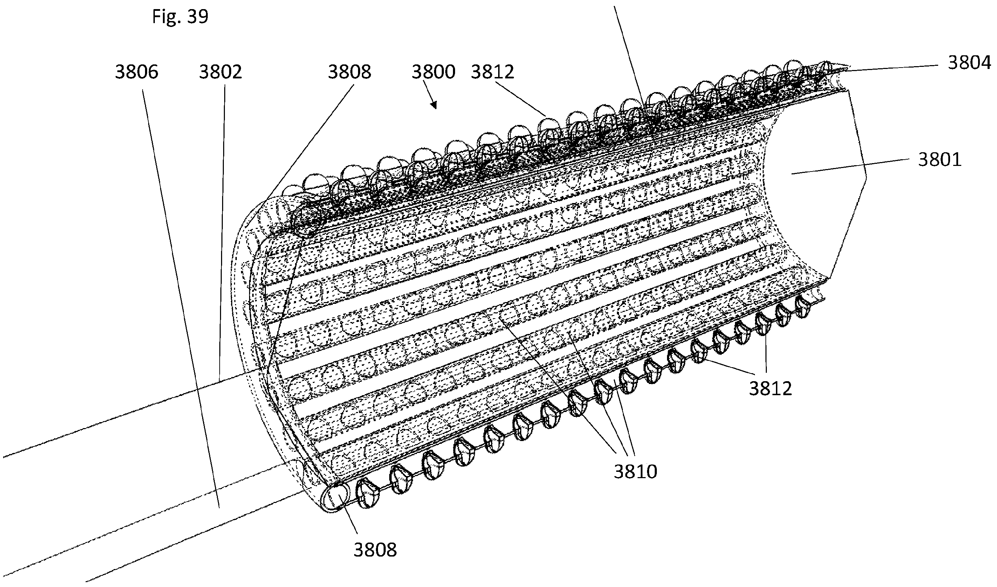

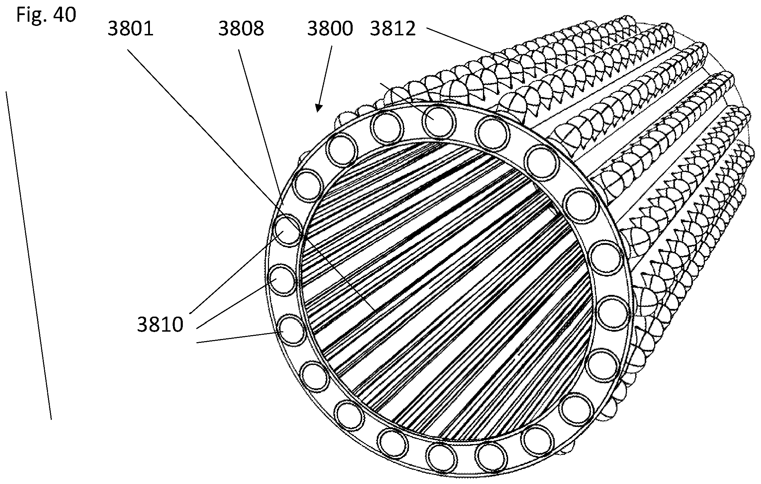

Reference is now made to Fig. 3, which is a simplified flow diagram that illustrates a third process for using treatment bodies according to the present embodiments. Initially, a patient is scanned 10 to located tumors. A treatment object having built-in expansion capability, for example according to any of the embodiments discussed in respect of Figs 22 to 40, is placed 12 in a placement device that allows medical personnel to make a precise location within the body. Depending on the embodiment, the object may be directly injected into the tumor or may be placed in a lumen and then fed to the tumor, or may be placed in tissue and fed to the tumor - 14. At the target location, which be on or in contact with the tumor, the object is released 16 and the object expands until it meets a preset pressure 30. A treatment protocol is applied 18. The treatment protocol may be a heating protocol, but may alternatively or additionally be a treatment protocol such as a chemotherapy protocol, involving the treatment object releasing drugs. The preset pressure is checked so that the object expands into the contracting tumor.

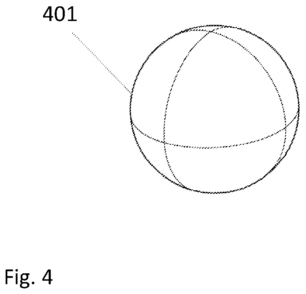

Reference is now made to Figure 4, which is a simplified diagram of a treatment object according to an example embodiment. Figure 4 illustrates a treatment object 401. A magnetic field source may provide pulses to move the treatment object 401 in steps through a medium (not shown), optionally a viscous medium, thereby to locate the treatment object 401 at a desired location in the medium. Positioning may for example be to an accuracy of 0.6mm or less, including 0.1mm. Movement may also be continuous.

The magnetic field source may induce heating in the treatment object 401. The source may be electro-magnetic and bring about induction. The source may be RF or may be micro-waves or any source of radiation that may cause heating. The treatment object 401 may be plastic and include magnetic particles within the plastic.

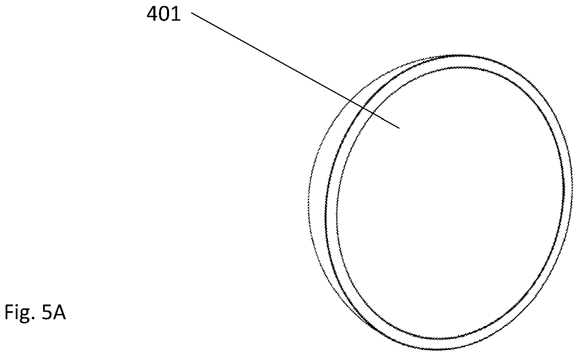

Reference is now made to Figure 5A, which is a simplified diagram showing an optional example embodiment of an interior of the treatment object of Fig. 4. As shown in Fig. 5A, the treatment object 401 may be hollow. The hollow interior may be used to ferry a therapeutic substance to a location to be treated.

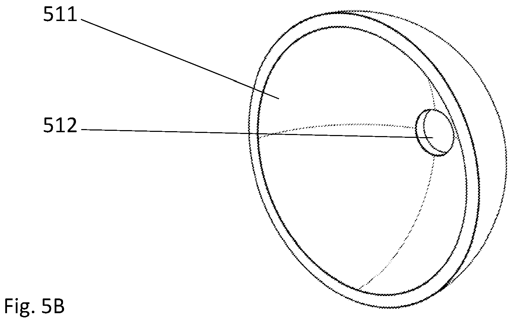

Reference is now made to Figure 5B, which is a simplified diagram showing an interior of a treatment object according to an example embodiment. As shown in Fig. 5B, a treatment object 511 may be hollow, and may optionally include a hole 512 in the wall of the treatment object 511. The hollow interior may be used to ferry a therapeutic substance to a location to be treated. The hole 512 may enable medication to exit the treatment object, and/or a sample from a patient's body to enter the treatment object 511.

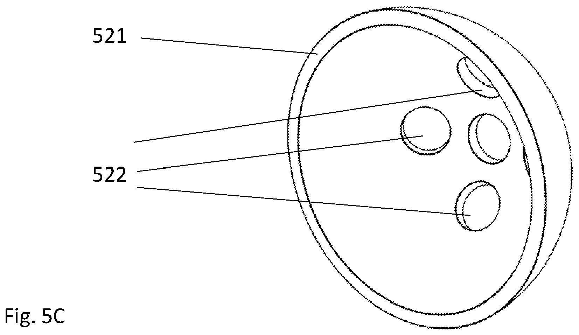

Reference is now made to Figure 5C, which is a simplified diagram showing an interior of a treatment object according to an example embodiment. As shown in Fig. 5C, a treatment object 521 may be hollow, and may optionally include more than one hole 522 in the wall of the treatment object 521. The hollow interior may be used to ferry a therapeutic substance to a location to be treated. The holes 522 may enable medication to exit the treatment object, and/or a sample from a patient's body to enter the treatment object 521.

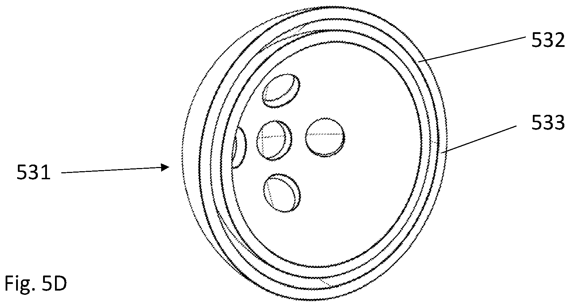

Reference is now made to FIG. 5D, which is a simplified diagram showing a magnetic layer with holes covered by a shell according to an example embodiment. As shown in Fig. 5D, a treatment object 531 may include a shell 532 of material over a magnetic layer 533. In some embodiments, the shell 532 is optionally dissolvable or melt-able to release a therapeutic substance. In some embodiments, the magnetic layer 533 optionally has holes, so that the therapeutic substance can leave the treatment object 531 once the shell 532 has dissolved or has been otherwise removed.

The shell 532 may be dissolved by receipt of a signal from a magnetic field source, thereby to ensure that release takes place at the target.

For example the shell 532 may be dissolved after a certain time has passed or by induction from the magnetic field source causing heating.

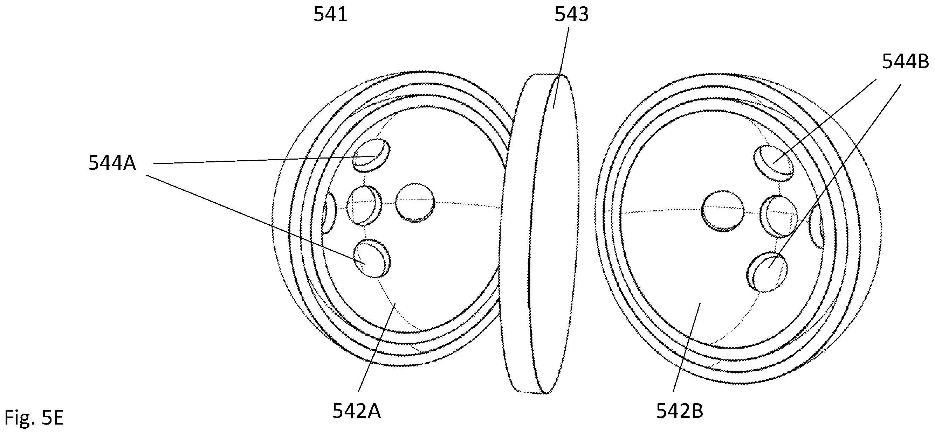

Reference is now made to FIG. 5E, which is a simplified diagram showing a variation in which two separate internal containers are available according to an example embodiment. As shown in Fig 5E, an interior of a treatment object 541 may be divided into two or more chambers 542a 542b by a central divider 543. Release of therapeutic substance may be at different rates as each chamber may have a shell that dissolves at a different rate. Alternatively one or more hole(s) 544a 544b may be of different sizes in the different chambers.

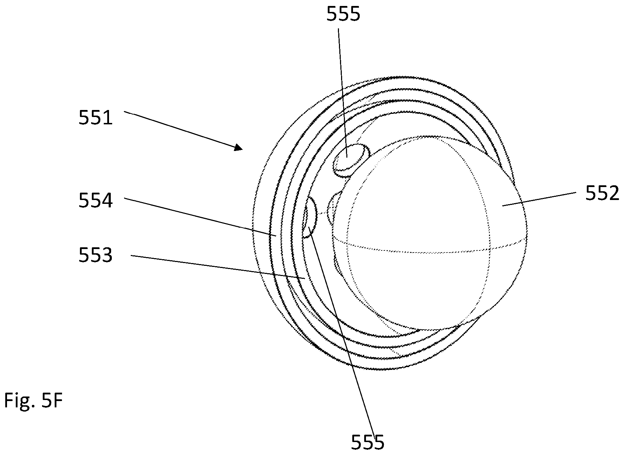

Reference is now made to FIG. 5F, which is a variation of the treatment object of Fig. 1. As shown in Fig. 5F, a treatment object 551 may have a central magnetized core 552 with a hollow shell 553 around about. In some embodiments, the hollow shell 553 may contain a therapeutic substance (not shown).

In some embodiments, the hollow shell 553 may be surrounded by an outer shell 554, which may optionally control release of a therapeutic material, optionally through optional hole(s) 555, similarly to the description above.

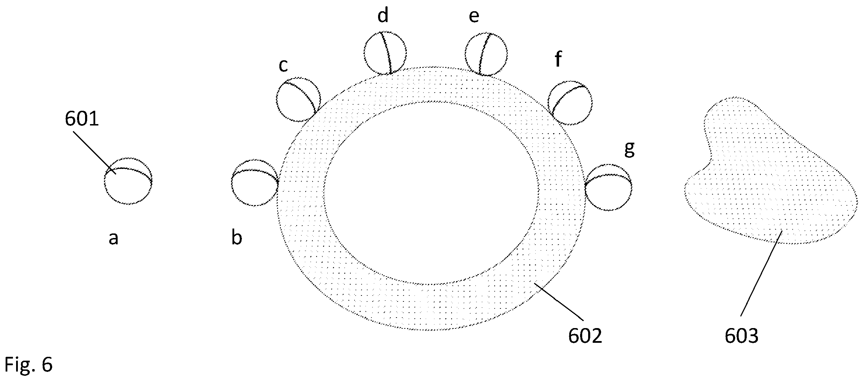

Reference is now made to FIG. 6, which is a simplified diagram showing a treatment object being moved around by pulses according to an example embodiment. Fig. 6 shows a treatment object 601 being moved in incremental steps a...g around a bone 602 to arrive at a tumor 603, optionally for delivery of treatment.

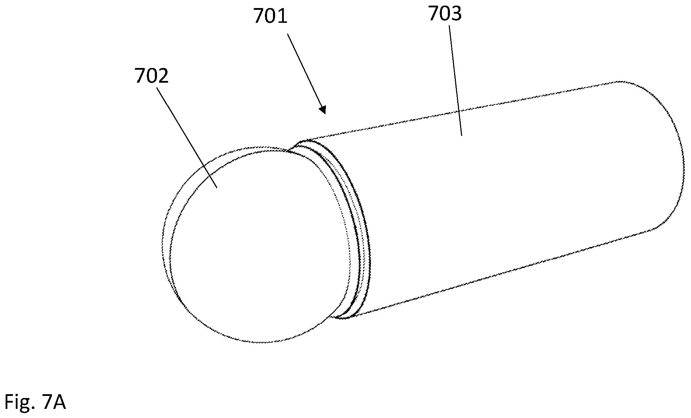

Reference is now made to FIG. 7A, which is a simplified diagram showing an example embodiment including a container part and a magnetized body. Fig. 7A shows a treatment object 701 with a magnetic section 702 and a tank section 703. In some embodiments, the tank section 703 is optionally empty and is opened at a desired location. A magnetic field source optionally provides a pulse to move the opened tank 703 further through the viscous medium (not shown) so that the empty tank section 703 is optionally filled with a sample of the viscous medium at the target location. In this way a sample can be taken of, say, a suspected tumor, to use in a biopsy. The hollow tank section 703 may be opened and then reversed out of a patient's body. In some embodiments, the tank section 703 may be filled with a material that melts or dissolves away. In some embodiments, once the filling material has melted away the tank section 703 may optionally be moved to capture material from the target area.

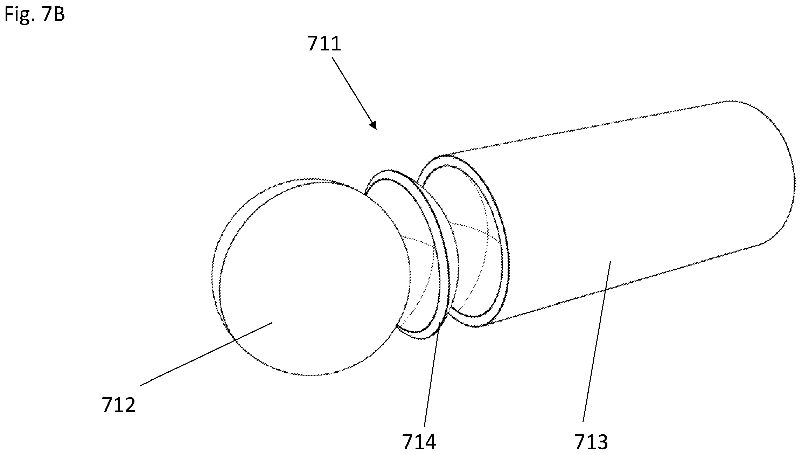

Reference is now made to FIG. 7B, which is a simplified illustration of a treatment object 711 variation of the embodiment of Fig. 7A. Fig. 7B is a variation of Fig. 7A showing a connection plate 714 that allows a tank section 713 to be separated from a magnetic section 712.

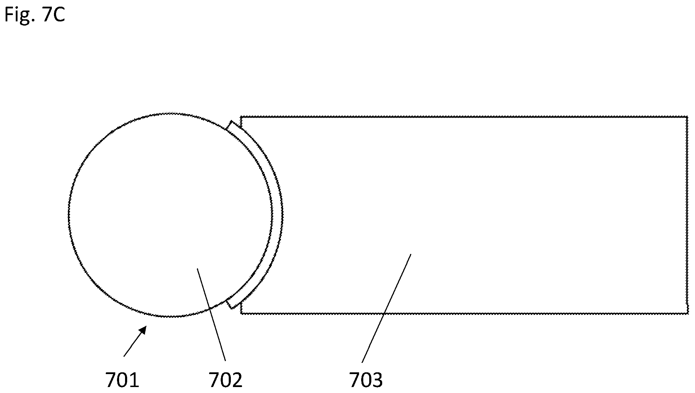

Reference is now made to FIG. 7C, which is a cross sectional view of the embodiment of Fig. 7A. Fig. 7C shows the treatment object 701 with the magnetic section 702 and the tank section 703 in cross section.

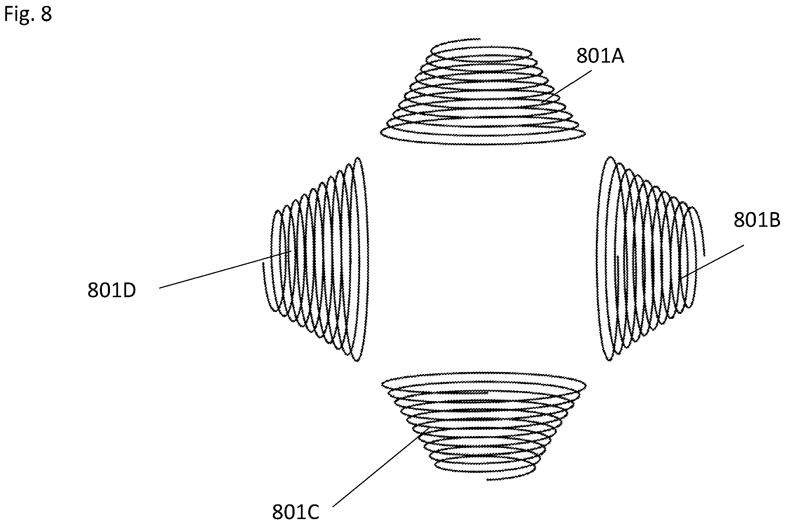

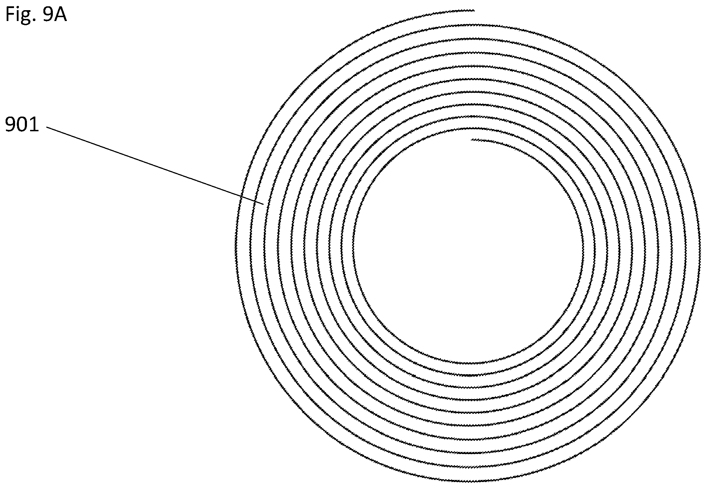

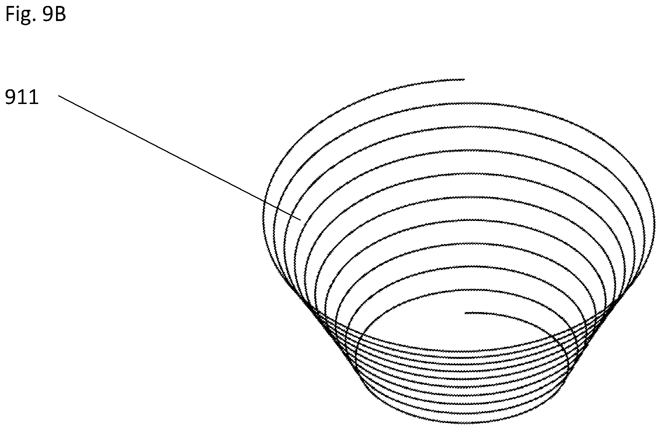

Reference is now made to FIGs. 8, 9A and 9B, which are examples of magnetic coils that may be used according to some example embodiments. Figs. 8, 9A and 9B show different magnetic coils that may be used to induce magnetic fields around a treatment object (not shown) to move the treatment object and/or induce heating in the treatment object. In some embodiments, multiple magnetic coils may be used at different locations, and their magnetic field intensities or locations may be changed in order to cause the magnetic object to move and navigate to a target location.

Fig. 8 shows several magnetic coils 801A 801B 801C 801D, optionally above and below a space where a treatment object is to be controlled, and/or optionally on both sides of the space where the treatment object is to be controlled.

In various embodiments, magnetic coils may be flat, cylindrical, conical, and various additional shapes as known in the art.

Fig. 8 shows conical magnetic coils.

Fig. 9A shows a flat magnetic coil 901.

Fig. 9B shows an isometric view of a conical magnetic coil 911.

In some embodiments, a method of targeting a location in a human or animal for treatment may include:
Inserting a treatment object into a human or animal;
Using a magnetic field to pulse the treatment object in incremental steps towards a treatment target;
Using x-ray based or other imaging to trace progress of the treatment object through the body; and
When the treatment object is at a desired location, activating the treatment object to apply a treatment.

Activating may involve heating, or dissolving or otherwise removing a cover to release a therapeutic substance.

An approach toward a treatment target may involve repeatedly approaching the target from different sides and applying the treatment at one or more or all of the approaches. **In** some embodiments, the treatment may be to remove tumors. **In** some embodiments, the tumor may be approached from various sides to destroy the tumor blood supply, thus inducing necrosis of the tumor. **In** case of a bone tumor the treatment object may be pulsed against the tumor multiple times. **In** the case of a blood clot the device may arrive at the blood clot and dissolve the clot. **In** the case of blood not clotting, the object may be delivered to a location to induce clotting. The device may be delivered to the location of gallstones and the like to destroy the stones.

As a further option, the treatment object may comprise a radioactive material, and be left at the target for a preset amount of time to provide a particular radiation dose. The treatment object may then be removed after the dose is completed.

In some embodiments, a processor may be used to obtain information from an imaging system and may optionally maneuver the treatment object. Alternatively or additionally a joystick may be used to guide the treatment object, where the movement of the joystick is optionally translated into electromagnetic pulses to navigate.

In some embodiments, under activation, the size and thermal capacity of the treatment object is known so the amount of heat to be applied can be precisely defined according to a desire to achieve specific damage to the treatment target.

Precise temperature levels can be achieved by changing the magnetic fields around the object, so as to cause local damage to a target without damaging the surrounding tissues. That is to say, the damage may be precisely targeted.

As explained in the background, a major part of the treatment of cancer in the human or animal body involves the killing and removal of tumors. However once a cancer has started to spread around the body, the surgeon is faced with numerous tumors, some of which may be quite inaccessible and some of which may be quite small, say of millimeter scale diameter. **In** such cases surgery may be impractical, leaving radiotherapy and chemotherapy as the two common options. Radiotherapy may be aimed very precisely at the tumor and may take advantage of the fact that tumor cells, because they are always dividing, are more sensitive than normal cells. Chemotherapy also takes advantage of the fact that tumor cells are constantly dividing, but cannot currently be aimed very precisely at the tumor and hence has side effects which are particularly noticeable in healthy body cells that divide more often.

An aspect of some embodiments provides a treatment object for treatment of a tumor that can be placed precisely into or next to the tumor and may deliver a precisely measured dose of treatment. An injector may be used to place a treatment object into the tumor in accordance with a tumor location obtained, optionally using some form of imaging. The treatment object then delivers the treatment from within, or right next to, the tumor. In some embodiments, the amount of treatment is optionally set according to the size of the tumor to kill the tumor while doing minimal damage to surrounding healthy cells.

In some embodiments the treatment object is already in place in the tumor as a result of being used in an imaging process.

The treatment object may contain a measured amount of therapeutic substance which may be released. According to embodiments the rate of release may be preset and/or may be controllable.

In some embodiments the object may heat itself to a preset or controllable specific temperature to kill the tumor cells. Tumor cells which are constantly dividing are more susceptible to raised temperature than healthy cells.

In some embodiments, the treatment object is optionally connected via wires to an external power source. The wires may also include a connection for temperature measurement so that the temperature of the treatment object may be monitored and controlled.

In some embodiments, no wires are used. Instead the treatment object includes an antenna to pick up radiation for heating by induction.

In some embodiments, no wires are used. Instead the treatment object itself picks up radiation for heating by induction.

In some embodiments, the treatment object may decompose after a while in the body into harmless substances. In some embodiments, the treatment object is removed after use by means of minor surgery.

An aspect of some embodiments may thus relate to devices for the treatment of a tumor.

An aspect of some embodiments may thus relate to a treatment object.

An aspect of some embodiments may thus relate to an injector for the treatment object.

An aspect of some embodiments may relate to a system that is a combination of the injector and the treatment object.

An aspect of some embodiments may relate to a power and/or control source.

An aspect of some embodiments may relate to a method of treatment of a tumor in humans and animals or may relate explicitly to treatment of a non-human animal.

According to an aspect of some examples of the present disclosure there is provided a treatment object that is placeable within a tumor, and activatable when within the tumor to deliver a dose of treatment. In other embodiments there is provided a system and a method.

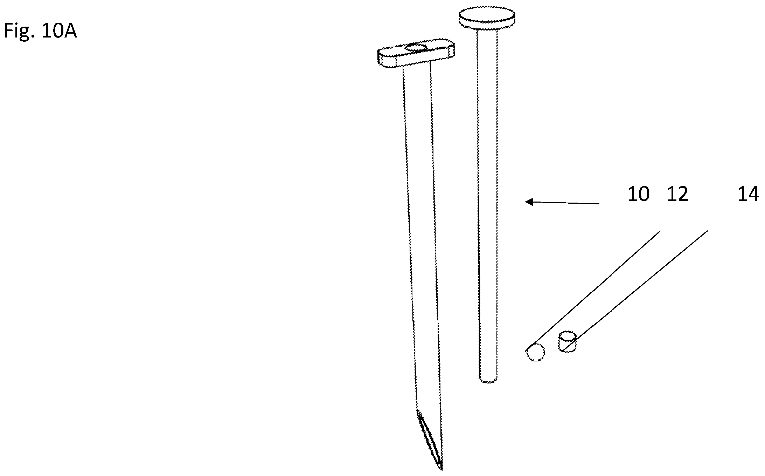

An aspect of some embodiment provides a treatment object that can be placed within a tumor, and is then activated when within the tumor to deliver a dose of treatment. The treatment object may be injected via needle into the tumor and may be powered via wire or by induction to deliver a controlled dose of a tumor treatment drug or of heat into the body of the tumor. In some embodiments, feedback may be provided on the treatment, and the feedback may be used to assess the treatment and/or determine the continuation of the treatment. Reference is now made to FIGs. 10A-E, which are simplified illustrations of an example embodiment of an injector and various example embodiments of treatment bodies.

Figure 10A illustrates an injector 10 and two different shaped example embodiments of treatment bodies 12 and 14 which may be injected by the injector 10 into a tumor.

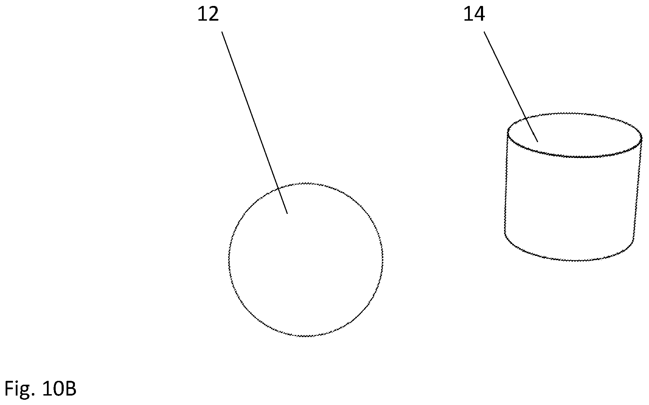

Fig. 10B shows a close-up of the two treatment bodies 12 and 14. The treatment bodies 12 and 14 are optionally placed within a tumor, and are able to optionally be activated when within the tumor to deliver a dose of treatment, as will be described in greater detail below.

As shown, the treatment bodies 12 14 are for placing in the injector 10, from which they can be ejected via an injector 10 needle to be placed precisely where intended, that is within a tumor of interest.

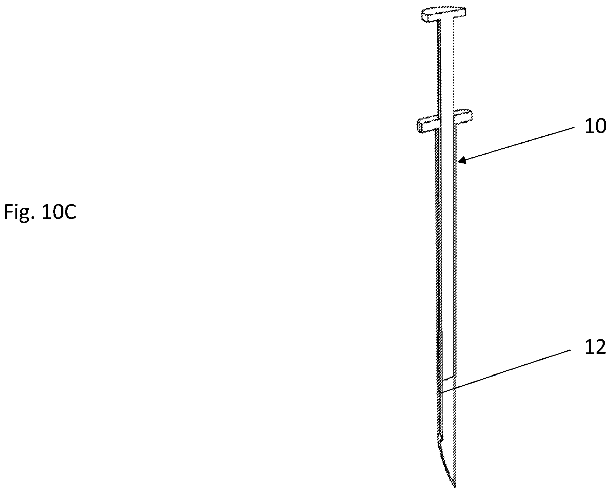

Fig. 10C shows a treatment object, for example one or more of the treatment bodies 12 14, in a cutaway of the injector 10.

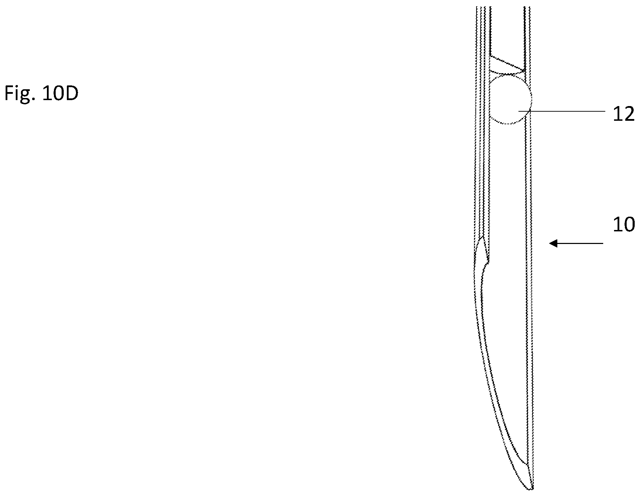

Fig. 10D is a detail of part of Fig. 10C with a treatment object, by way of a non-limiting example treatment object 12, inside the injector 10.

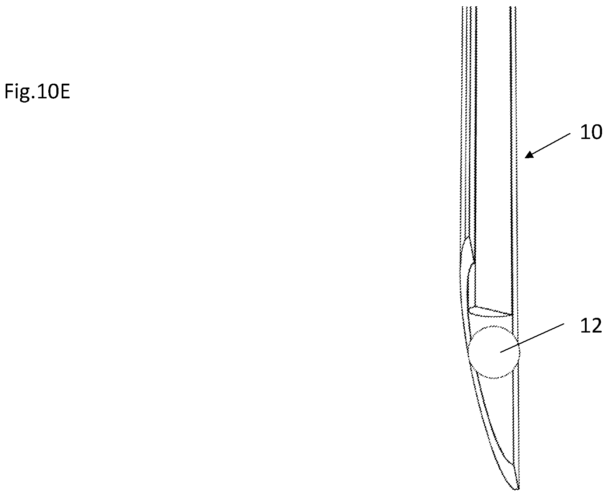

Fig. 10E shows the treatment object 12 being pushed out through a needle tip 16 of the injector 10 into a tumor (not shown).

In some embodiments, the injector 10 may come pre-fabricated with the treatment object 12 14 inside, ready for injection. In some embodiments, the injector 10 may be provided with different needle lengths according to the depth of the tumor. In some embodiments, the injector 10 may be provided with different needle diameters according to a diameter of the treatment object 12 14.

In some embodiments, a needle tip 16 of the injector 10 may be breakable or otherwise detachable, so that a treatment object, be it a treatment object such as treatment bodies 12 14 shown herein, or the needle tip 16 itself, is optionally placed in the tumor by detaching from the injector 10.

In some embodiments, an endoscope is optionally provided with or attached to the injector 10, to help with precise location on the tumor.

In some embodiments, a treatment object may be inserted into an empty injector and an endoscope may be supplied together with or separately from the treatment object and/or injector.

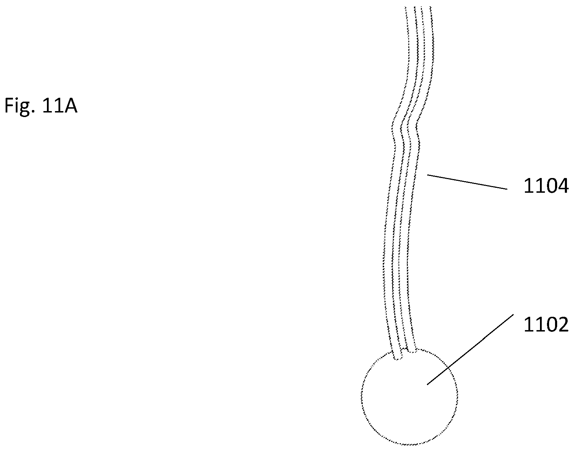

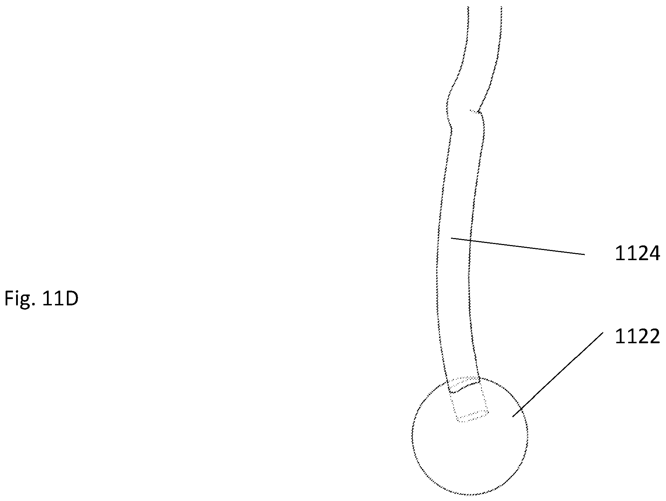

Reference is now made to FIGs. 11A-C, 12A-C and 13A-H, which are simplified illustrations of a treatment object attached via wires according to example embodiments.

Figs. 11A-C, 12 and 13A-H illustrate a treatment object attached to wires. The treatment object may be connected via the wires to a power supply and/or a controller and/or a sensor such as a temperature sensor, including to a combined power supply and controller.

Fig. 11A shows a treatment object 1102 attached to two wires 1104. In some embodiments, the two wires provide electric current to heat the treatment object 1102.

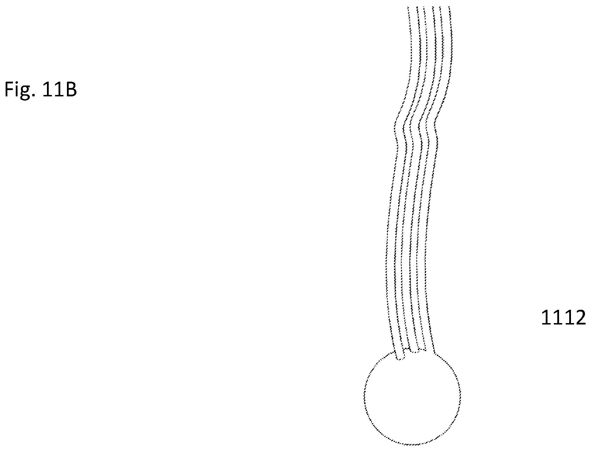

Fig. 11B shows a cross section of a treatment object 1112 attached to two wires 1114 and an optional third wire 1116 attached to an optional temperature sensor 1118, such as, by way of a non-limiting example, a thermistor.

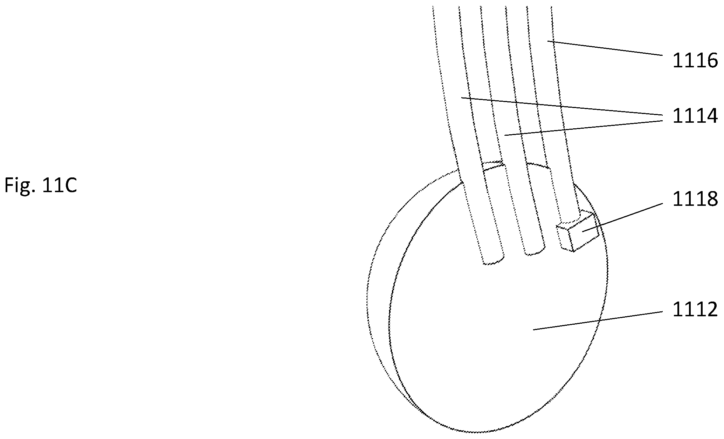

Fig. 11C shows a treatment object 1122 attached to a cable 1124 which includes one or more wires with the cable 1124.

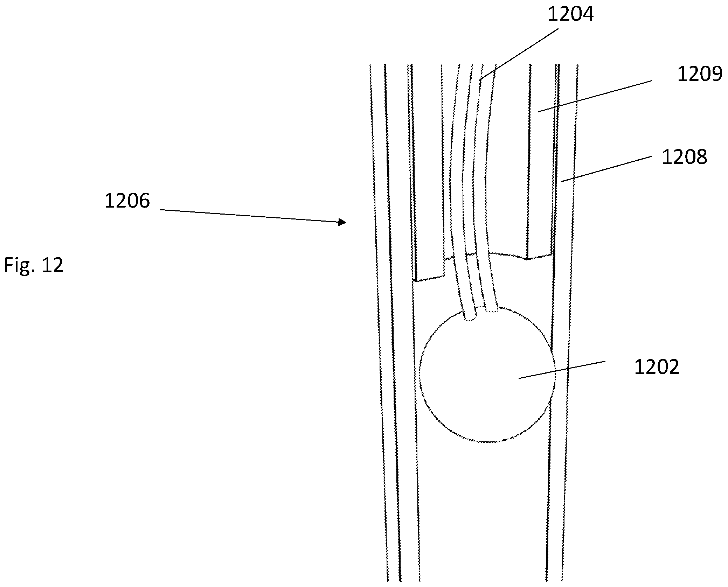

Fig. 12 shows a treatment object 1202 attached to two wires 1204 within an injector 1206. The injector optionally includes an outer sleeve 1208 and an inner plunger 1209.

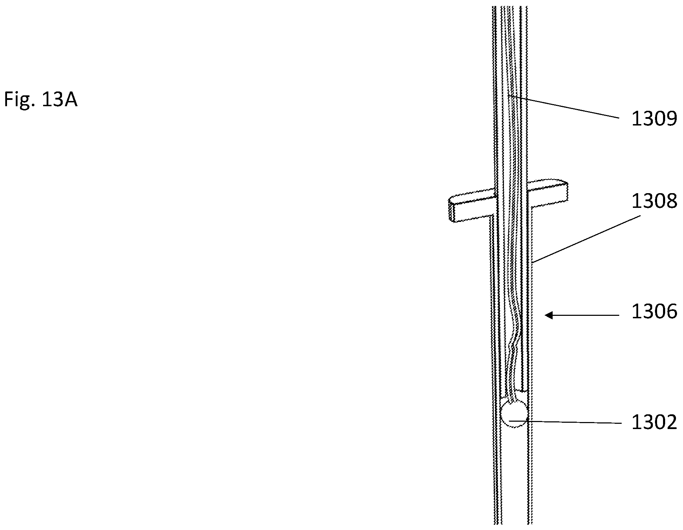

Fig. 13A shows a treatment object 1302 attached to wires (not referenced in Fig. 13A) within an injector 1306. The injector 1306 is shown with an inner plunger 1309 not fully pushed into an outer sleeve 1308 or needle 1308.

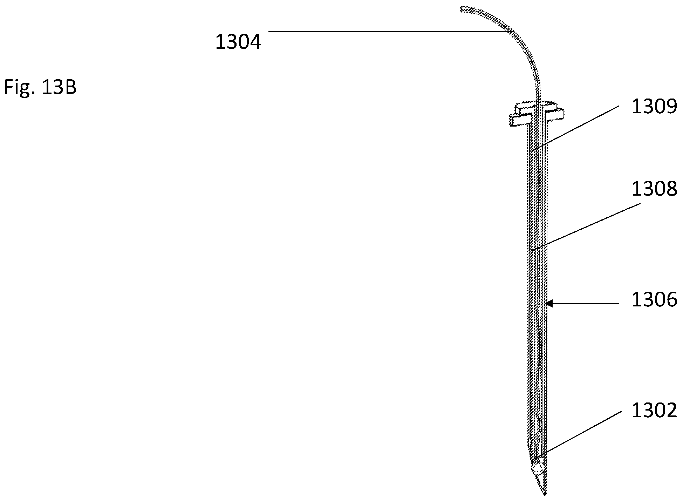

Fig. 13B shows the treatment object 1302 of Fig. 13A attached to wires 1304 within the injector 1306. The injector 1306 is shown with the inner plunger 1309 fully pushed into the outer sleeve 1308 or needle 1308.

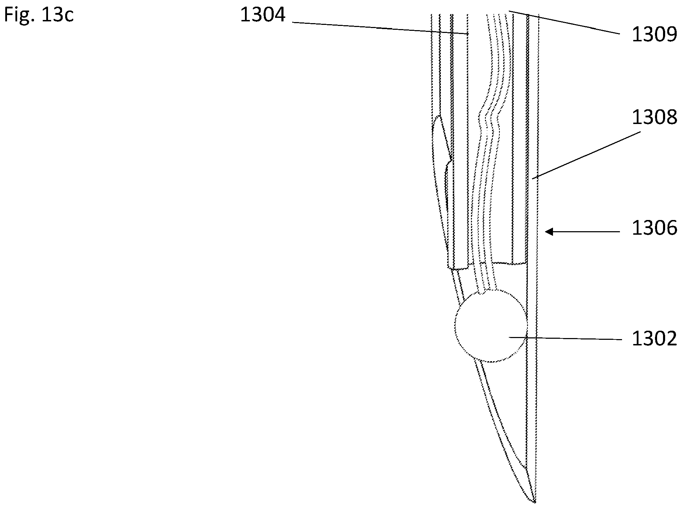

Fig. 13C shows the treatment object 1302 of Fig. 13B attached to the wires 1304 within the injector 1306. The treatment object 1302 is shown at a tip 1305 of the outer sleeve 1208 or needle 1208, having been pushed there by the plunger 1309.

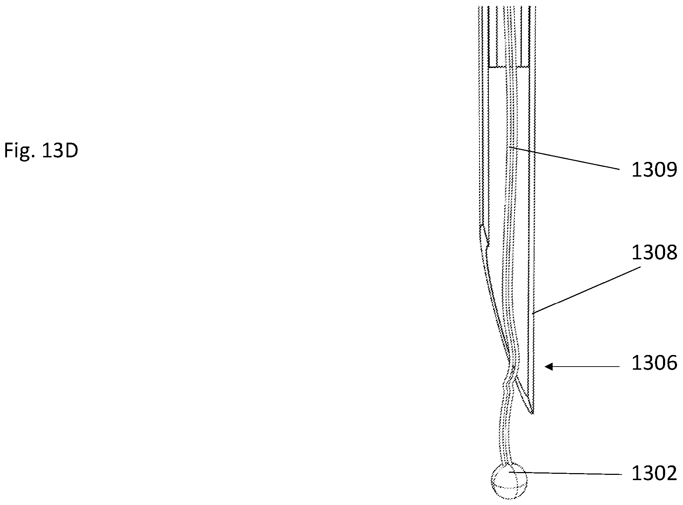

Fig. 13D shows the treatment object 1302 of Fig. 13C attached to the wires 1304, and extended outside the injector 1306.

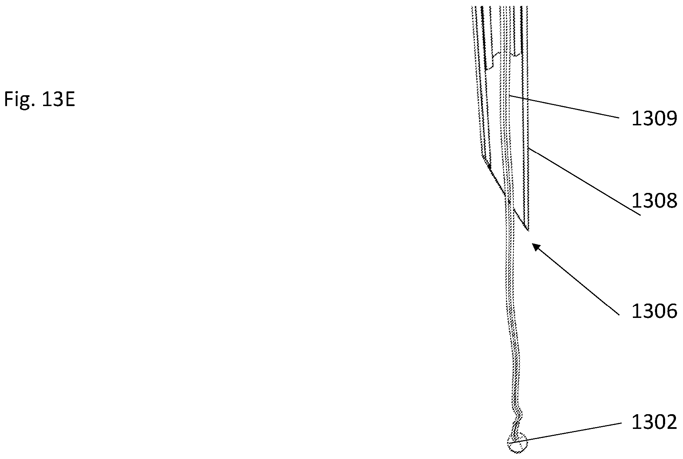

Fig. 13E shows an enlarged view of the treatment object 1302 of Fig. 13D attached to the wires 1304, and extended outside the injector 1306.

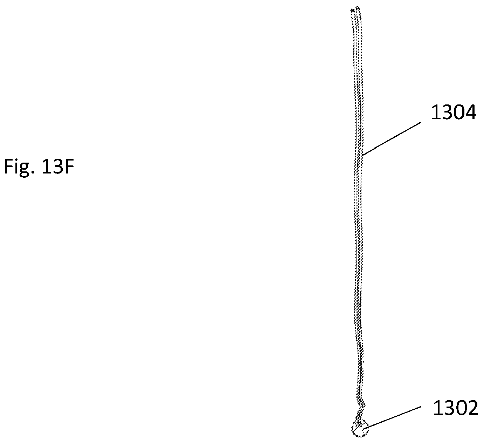

Fig. 13F shows the treatment object 1302 of Fig. 13D attached to the wires 1304, treatment object, without the injector 1306.

In some embodiments, the injector 1306 is optionally withdrawn from a patient's body, leaving the treatment object 1302 within the patient's body, and the wires 1304 leading from the treatment object 1302 to outside the patient's body.

In some embodiments, the treatment object may receive power via the wires.

The treatment object may release a predetermined treatment dose into the tumor by activation via the wires. That is to say a controller sends a signal to activate the treatment at the treatment object.

The treatment object may release a particular treatment into the tumor following activation via the wires and may send feedback regarding the dose via the wires. A controller may thus be able to monitor efficacy of the treatment and also direct the way in which the treatment is to continue.

The treatment object may controllably release a treatment into the tumor in response to signaling via the wires and may send feedback regarding the dose via the wires.

The treatment may involve releasing a substance for killing the tumor. As discussed elsewhere the substance may be released by opening a cover. In some embodiments, controlled release may be achieved by using a semi-permeable membrane.

The treatment may involve heat. The heat may be applied to the tumor. For example the treatment may involve heating the tumor such that boundaries of the tumor reach a predetermined temperature selected to cause tumor death with minimal effect on surrounding cells. In some embodiments, the heating may be for a set amount of time, optionally selected on the basis of the properties of the tumor to effectively kill the tumor.

The treatment of tumors using heat is known in the art as hyper-thermal treatment and is generally carried out from outside the body. As typically practiced, the heat is not well directed at the tumor, especially if it is deep within the tissues, and considerable amounts of energy miss the tumor and cause damage to surrounding tissue.

Some embodiments potentially alleviate the problem of damage to surrounding tissue by applying heat from within the tumor. In some embodiments, the heat is applied at amounts calculated for specific properties of a specific tumor. The properties of the tumor include one or more of mass, tissue type, level of hydration, density, proximity to blood vessels (which may dissipate heat), extent of necrosis in the tumor and/or adjacent tissue and processes induced by necrosis, quantity or percentage of fat in the tumor, level of salinity and size of the tumor.

In some embodiments, suitable temperatures may be, for example 42, 43, 44 and 45 degrees Celsius and a particular temperature of interest is 43.5 degrees Celsius.

In some embodiments, tumor temperatures, named herein Ttemp, are controlled so as to damage the tumor.

In some embodiments, the temperatures are selected as temperatures to be reached at extremities or periphery of the tumor, named herein TPtemp.

In some embodiments, the treatment object reaches a temperature, named herein Tbody, used so that at least a portion of the tumor reaches Ttemp, and/or so that the extremities or periphery of the tumor reach TPtemp.

In some embodiments, TPtemp is controlled not to exceed a specific maximal temperature, so as to alleviate or prevent damage to surrounding tissues.

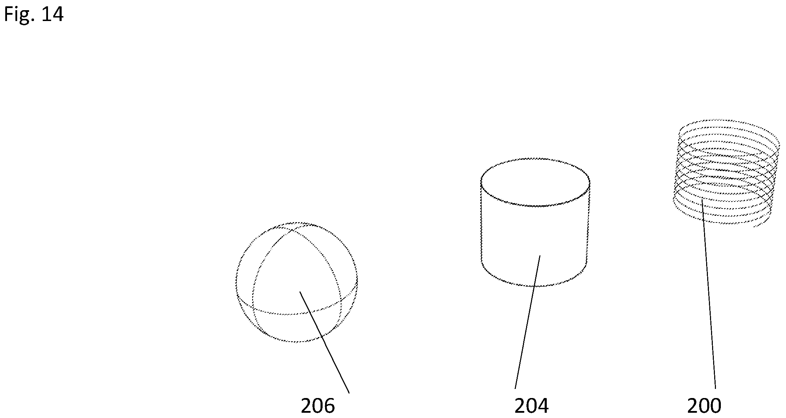

Reference is now made to FIG. 14, which is a simplified illustration of a treatment object incorporating an antenna according to examples of the present disclosure.

Fig. 14 shows an antenna 200 which may be incorporated into treatment bodies, to allow induction to wirelessly provide power to the treatment bodies. In some embodiments, the power causes the treatment bodies to heat. Two example embodiments of treatment bodies 204 206 are shown in Fig. 14.

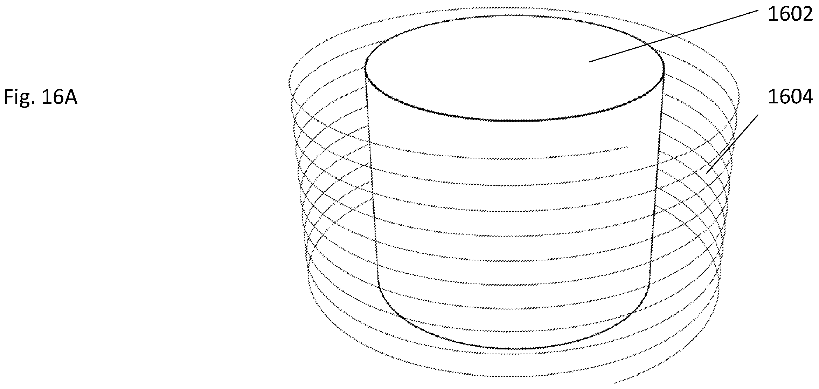

In some embodiments, the antenna 200 may be built into an outer skin of the treatment bodies to optionally form an outside of the treatment bodies, as shown in Figs. 16A-D.

In some embodiments, the treatment bodies may be hollow inside.

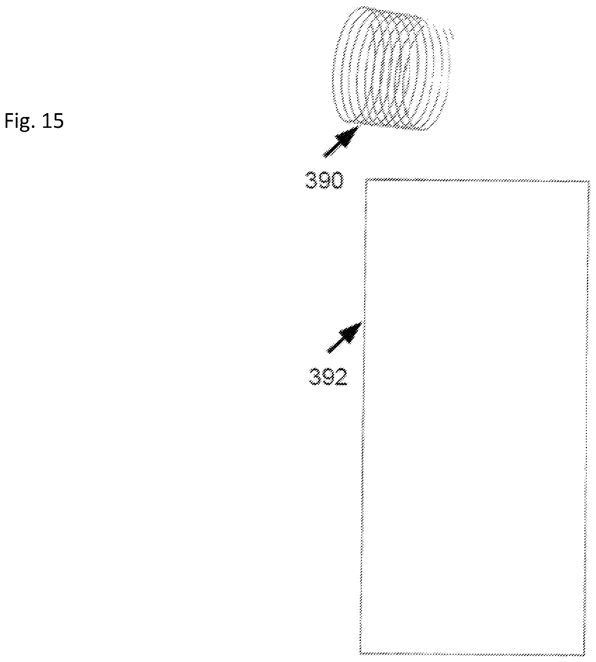

Reference is now made to FIG. 15, which is a simplified diagram illustrating an external antenna for providing energy for energizing the device of the present embodiments.

Fig. 15 shows a coil 390, held externally to a body 392, which may provide radiation at a given frequency, for example RF radiation, to be received by a treatment object within the body, to provide induction, and optionally, in some embodiments, heating.

The treatment object may thus be powered by induction. The induction powered treatment object may contain a predetermined amount of a substance for killing the tumor, in some embodiments optionally placed in a hollow in the treatment object, and may be inductively powered to release the substance into the tumor. An amount of the substance may be selected according to a size of the tumor. That is to say, the tumor is identified, and a treatment object is optionally filled with an amount in accordance with the specific tumor the treatment object is intended for.

In some embodiments, the treatment object may be heated by induction.

Reference is now made to FIGs. 16A-D, which are simplified illustrations of a treatment object incorporating an antenna according to examples of the present disclosure.

Fig. 16A shows an example embodiment of a treatment object 1602 surrounded by an antenna 1604.

In some embodiments, when the antenna 1604 receives transmitted energy, the antenna heats up, potentially heating the treatment object 1602, causing the treatment object 1602 to affect a tumor.

Affecting the tumor, in various embodiments, may be achieved by one or more of:
Heating the tumor;
Melting an envelope surrounding the treatment object, enabling the treatment object to release medication to treat the tumor; and
Causing a door or flap in the treatment object to open or melt, enabling the treatment object to release medication to treat the tumor.

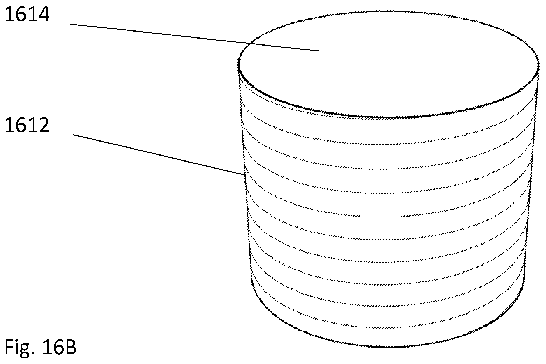

Fig. 16B shows an example embodiment of a treatment object 1612 into which is included, or embedded, an antenna 1614.

Operation of the treatment object 1612 may be similar to the operation described above with reference to the treatment object 1602 shown in Fig. 16A.

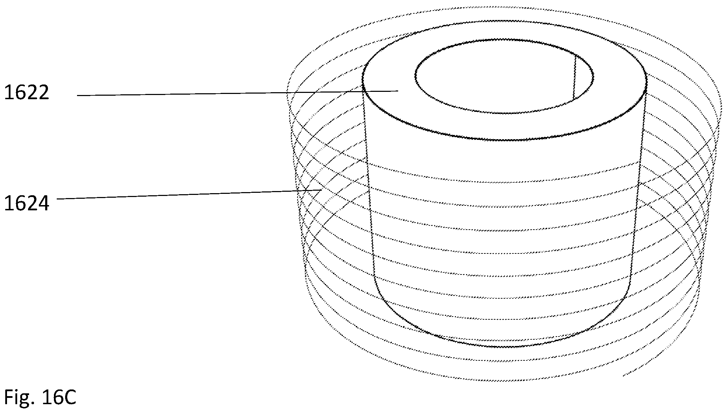

Fig. 16C shows an example embodiment of a treatment object 1622 shaped as a hollow cylinder, surrounded by an antenna 1624.

Operation of the treatment object 1622 may be similar to the operation described above with reference to the treatment object 1602 shown in Fig. 16A.

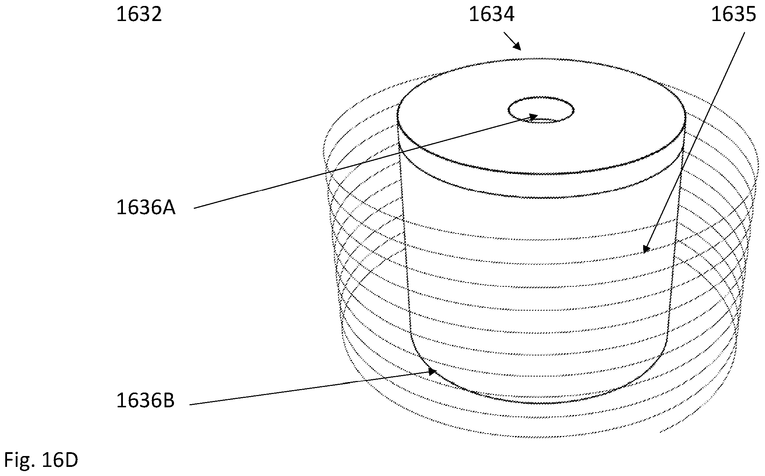

Fig. 16D shows an example embodiment of a treatment object 1632 shaped as a cylinder with a hollow 1635 within, with one or more opening(s) 1636A 1636B in at least one end of the cylinder, surrounded by an antenna 1634.

Operation of the treatment object 1632 may be similar to the operation described above with reference to the treatment object 1602 shown in Fig. 16A.

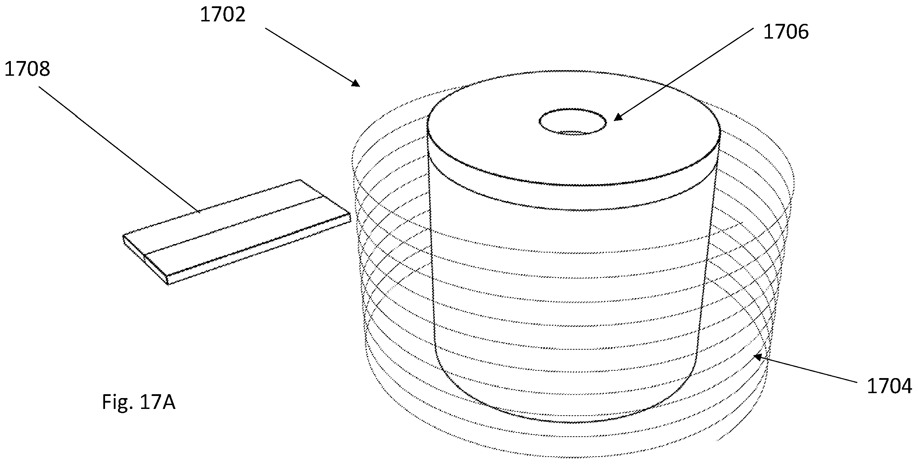

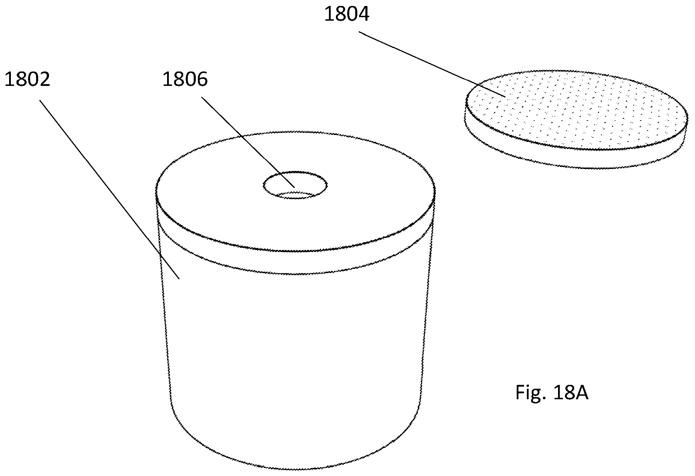

Reference is now made to FIGs. 17A-F and 18A-B, which are simplified illustrations of various ways in which a cover may be added to a treatment object according to example embodiments. In some embodiments, a treatment object may include an openable cover, which is opened by heating to release medication, as shown in Figs 17A-E and 18A-B.

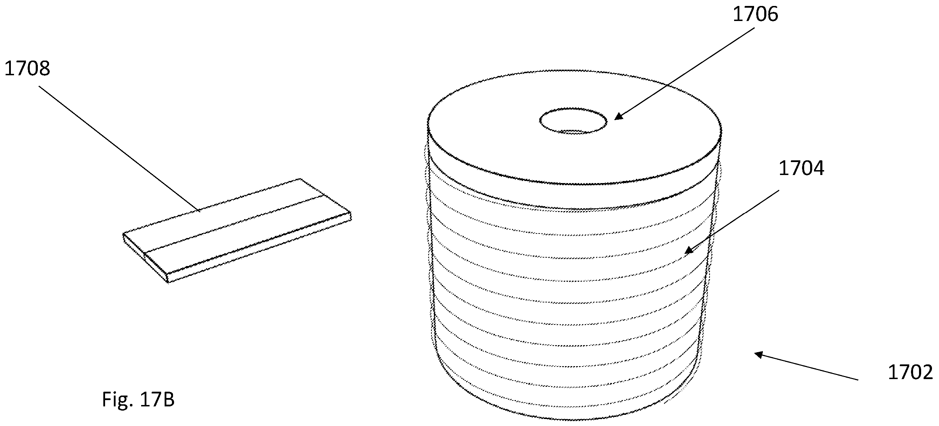

Figs. 17A and 17B show a hollow treatment object 1702, with an optional antenna 1704. The hollow treatment object 1702 has a hole 1706 at one end. Such a hole 1706 is optionally covered and closed by a cover 1708. The antenna is loosely wrapped in Fig. 17A and tightly wrapped in Fig. 17B

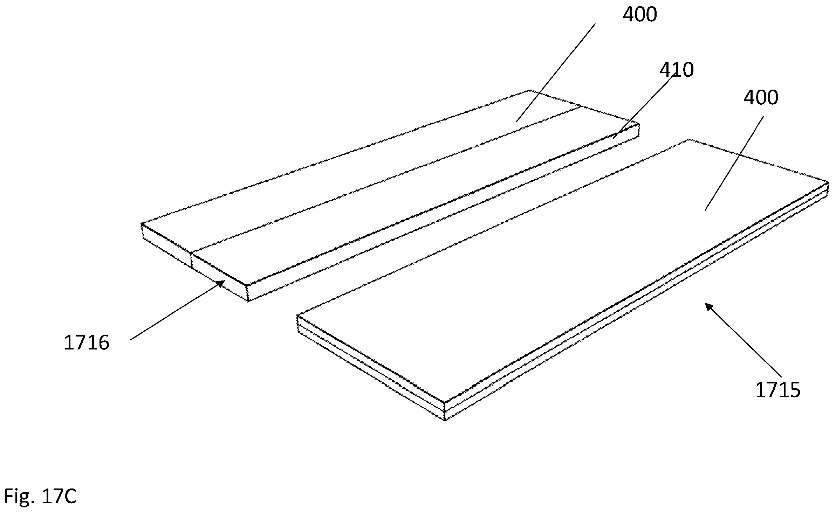

Fig. 17C shows two example embodiments of covers 1715 1716

The covers 1715 1716 may optionally be made of two materials 400, 410 having different heating coefficients so that they expand at different rates causing a cover to bend and thus open and release a substance contained within the treatment object 1702.

In some embodiments, the treatment object 1702 may act as a heating element within a tumor to deliver a dose of heating to kill the tumor from within. The temperature is selected as discussed herein.

In some embodiments, delivery of substance and acting as a heating element may be combined into a single device.

In some embodiments, the temperature may be altered by changing a frequency of induction.

In some embodiments, the temperature of the treatment object may be regulated by setting intervals at which heating is applied and intervals at which heating is not applied, that is to say by providing a duty cycle.

In some embodiments, an amount of energy needed to kill the tumor may be set according to the nature of the tumor, by way of a non-limiting example by estimating an amount of hydration and size of the tumor.

In some embodiments, the amount of heat is optionally determined based on heat dissipation of the tissue. By way of a non-limiting example, one tumor may be more rapidly cooled due to being close to a blood supply, whereas another tumor may not have much in the way of large blood vessels or a large number of blood vessels in the vicinity of the tumor.

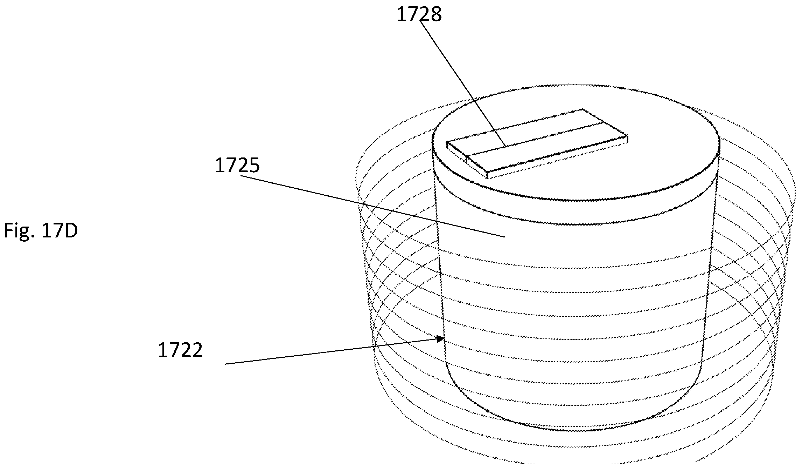

Fig. 17D shows a hollow 1725 within a treatment object 1722, with an optional antenna 1724. The hollow 1725 within the treatment object 1722 has a hole at one end. Such a hole is optionally covered and closed by a cover 1728.

Opening of the cover 1728 of the opening of the hollow 1725 treatment object 1722 may be as described above with reference to Figs. 17A and 17B.

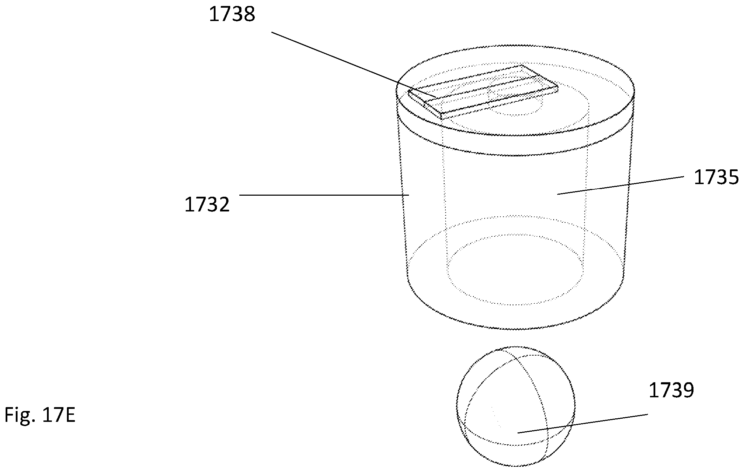

Fig. 17E shows a hollow 1735 within the treatment object 1732.

In some embodiments, the hollow 1735 with the treatment object 1732 optionally has a hole at one end. Such a hole is optionally covered and closed by a cover 1738.

Opening of the cover 1738 of the opening of the hollow 1735 treatment object 1732 may be as described above with reference to Figs. 17A and 17C.

In some embodiments, the hollow 1735 within the treatment object 1732 has a hole at one end. Such a hole is optionally plugged by a plug 1739.

In some embodiments, the plug 1739 is optionally configured to be heated, by way of a non-limiting example by receiving RF radiation. In some embodiments, the plug 1739 separates from the treatment object 1732 when heated, by way of a non-limiting example by melting glue attaching the plug to the treatment object 1735, or by melting the substance of the treatment device 1735. In some embodiments, separating the plug 1739 from the treatment device 1735 enables a substance stored within the treatment object 1735 to exit the treatment object 1735 and affect a tumor.

In some embodiments, one or more of the plug 1739 and the treatment object are optionally soluble in a patient body.

In some embodiments, one or more of the plug 1739 and the treatment object are optionally insoluble in a patient body.

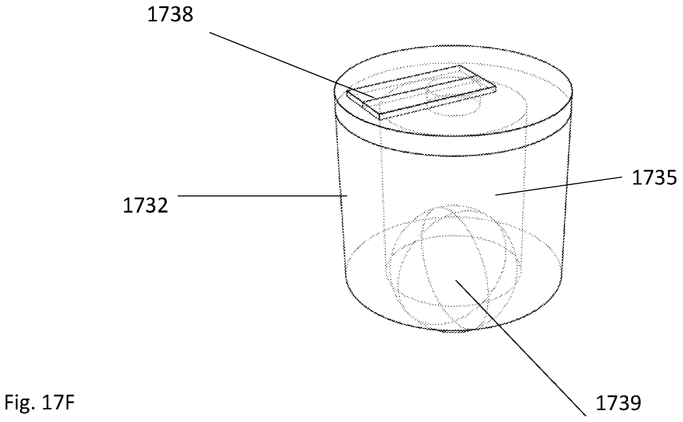

Fig. 17F shows the hollow 1735 treatment object 1732 plugged by the plug 1739.

Fig. 18A shows a hollow treatment object 1802, with an opening 1806 on one end of the treatment object 1802, and with an optional cover 1804.

In some embodiments, the hollow treatment object 1802 has a hole 1806 at one end. Such a hole 1806 is optionally covered and closed by the cover 1804.

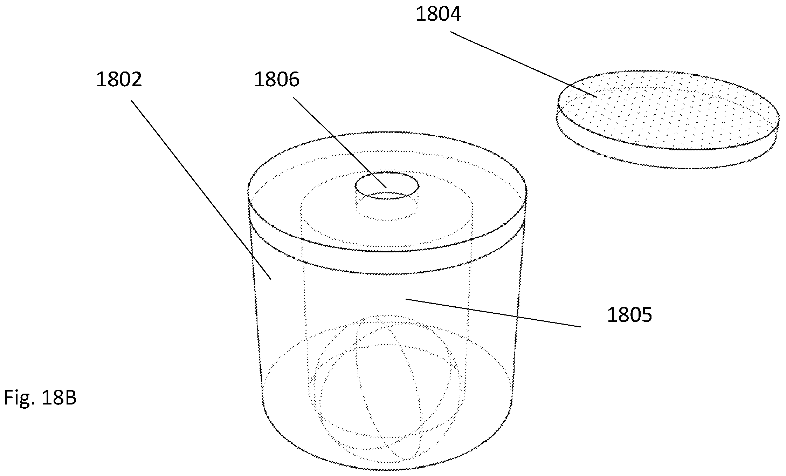

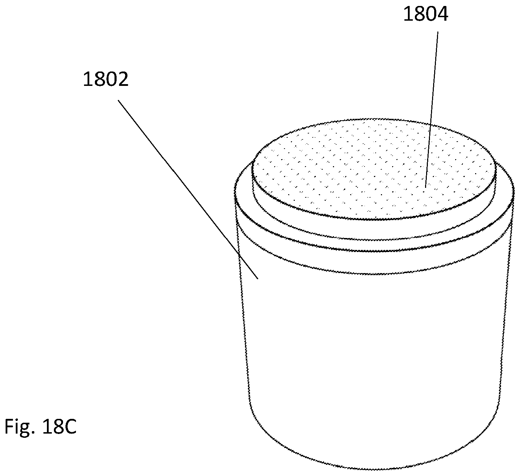

Fig. 18B shows the hollow 1805 within the treatment object 1802, and Fig. 18C shows the cover 1804 covering the opening 1806.

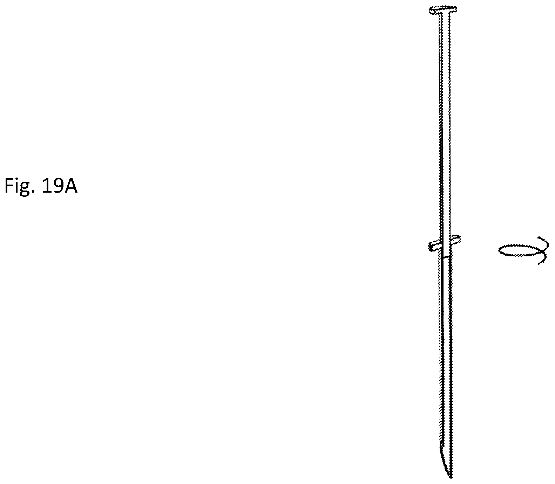

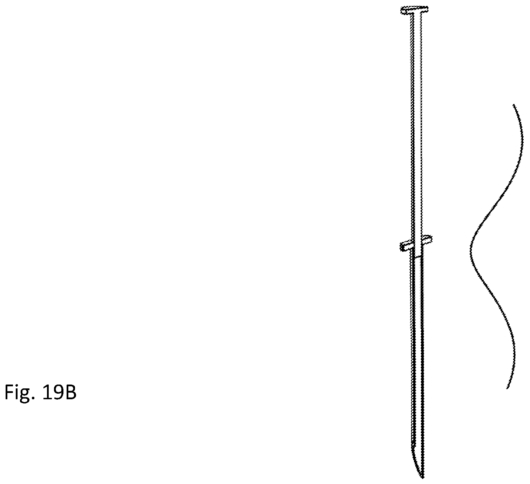

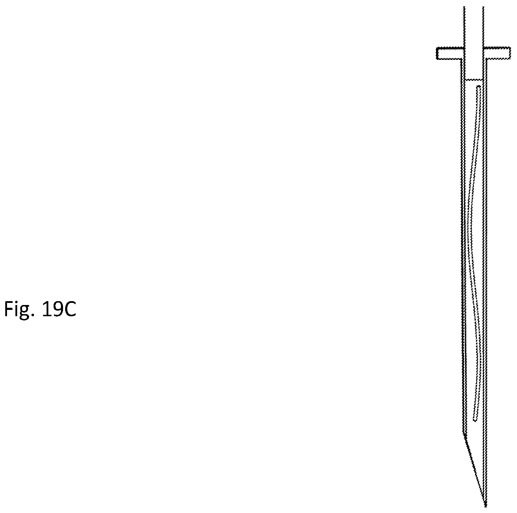

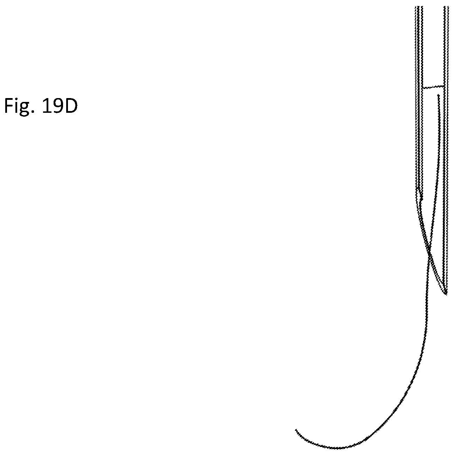

Reference is now made to FIGs. 19A-F, which are simplified illustrations of a treatment object including a wire antenna according to example embodiments.

In some embodiments, a treatment object includes an antenna, configured to be inserted into a body via a tube such as a syringe, needle, or injector as described herein or as are known in the art.

In some embodiments, the treatment object is an antenna, configured to be inserted into a body via a tube such as a syringe, needle, or injector as described herein or as are known in the art.

In some embodiments, the antenna is optionally a wire which, when released from an injector, assumes a specific shape.

In some embodiments, the wire is a tensioned wire, which, when released from the injector, assumes a specific shape. In some embodiments, the wire is a pre-tensioned wire, which, when released from the injector, assumes a specific shape.

In some embodiments, the wire is made of shape-memory material, which, when released from the injector, assumes a specific shape.

Figures 19A to 19D illustrate example embodiments in which a treatment object includes a tensioned wire, the tension determining a selected shape to be assumed by the wire when released. The wire is given a shape and then straightened out to fit into an injector. As the wire is released into the tumor the wire returns to its given shape, thus providing a heating element, or a drug release device, or both, preferable at a location of a tumor and/or extending around the tumor.

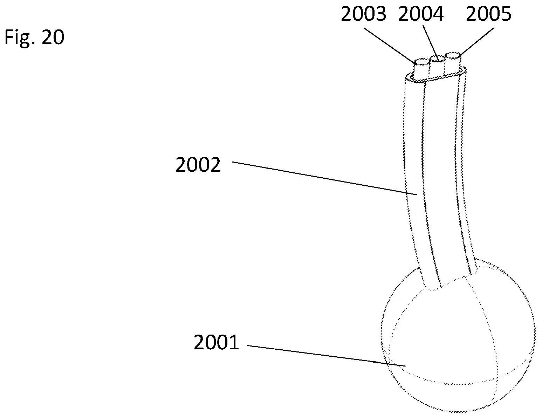

Reference is now made to Fig. 20, which is a simplified diagram showing a treatment object 2001 connected to a sheath 2002. The sheath holds multiple separate wires or fibers or tubes 2003, 2004 and 2005. Thus in an embodiment of an electrically powered device, they are wires, and two of them may be the positive and negative wires, the third being a signal wire to a thermistor for thermostatic control. In an embodiment in which a laser energy source is used, they may be one or more optical fibers, one optical fiber for providing energy for heating and possibly a second fiber for optical signaling with a thermistor. In an embodiment one may be a hollow tube providing measured doses of a treatment substance. In another embodiment, the tubes may be forward and return channels for a heating fluid. The heating fluid may for example be heated at an antenna that is placed internally within the patient. In an embodiment, one of the wires may lead to a thermistor or other temperature measuring element allowing for temperature based feedback and thermostat control, to keep the device safe.

**In** some embodiments, advantage is taken of markers that may have been left in a body, possibly in a tumor, by previous imaging or other diagnosis procedures. An example embodiment method of treating a tumor is provided that includes ascertaining that a previous imaging process has left a marker, possibly a metal marker, in a tumor. **In** some embodiments, it is ascertained how much energy is needed to kill the tumor based on the properties of the tumor to absorb heat and on properties of the marker to be susceptible to induction and a heating effect. Some embodiments may use induction to heat the marker, and apply the required amount of energy needed to kill the tumor.

In some embodiments, markers which are conducive to heating by absorption of radiated energy are optionally provided for imaging or other diagnosis procedures, for potential eventual use in heating and affecting a tumor.

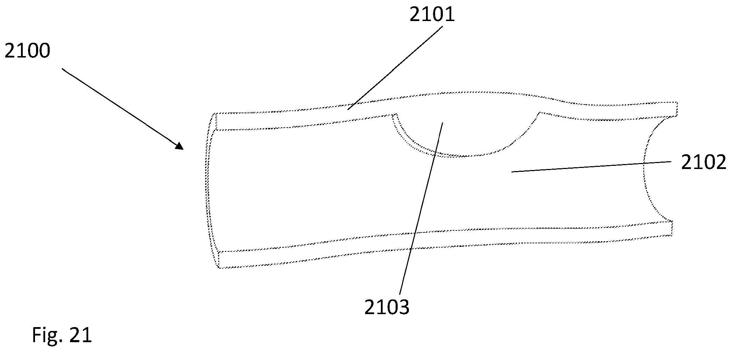

Reference is now made to Fig. 21, which is a simplified diagram showing a body organ 2100 such as the stomach, the large or small intestine or the colon, in which an organ wall 2101 surrounds a lumen 2102. A tumor 2103 appears in the wall 2101. Typically, in the existing art, such a tumor would be removed by surgery. However it is not always easy to access.

Reference is now made to Figs. 22A and 22B. A treatment object 2200 is in this embodiment built like a stent in which a spring-like construction has a closed position in which the body is moved along the lumen of organ 2100 to arrive at the position of the tumor 2103. As shown in Fig. 22B, the treatment body 2200 comprises outer spring-like structure 2202 over a bladder 2204. The bladder can be inflated via valve 2206 and tube 2208 under the spring-like structure. Fig. 22C is an exploded diagram showing the upper and lower parts of the spring-like construction 2202 over the bladder 2204. As shown in Fig. 22D the treatment object 2202 is maneuvered centrally within the lumen 2100 to a location under the tumor 2103. As shown in Fig. 22E the bladder 2204 is expanded to bring the spring-like structure into contact with the tumor 2103. The spring-like structure is heated, using any of the methods discussed elsewhere herein to kill the tumor cells in immediate contact. The heated tumor cells die and the bladder is further inflated to retain pressure. Further tumor cells die and the bladder expands further, until the tumor withers away.

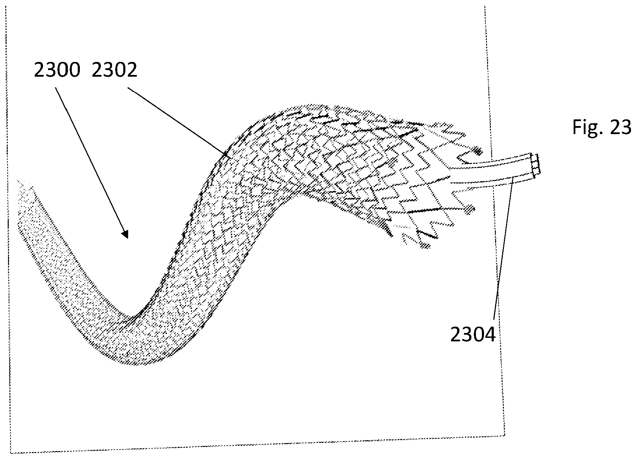

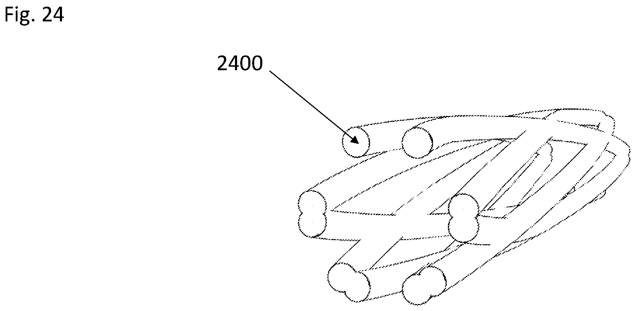

Reference is now made to Fig. 23, which shows an elongated treatment object 2300 comprising a spring-like outer structure 2302 and a bladder 2304 for expansion. The elongated object may be useful for numbers of tumors around a central space, such as tumors of the gut and colon that have started to spread. Fig. 24 shows a detail 2400 of the spring-like outer structure. In embodiments, the bladder may be removed after expansion of the spring-like outer structure so as not to obstruct the organ. In other cases, where the object is placed inside a tumor but obstruction is not an issue, the bladder may remain.

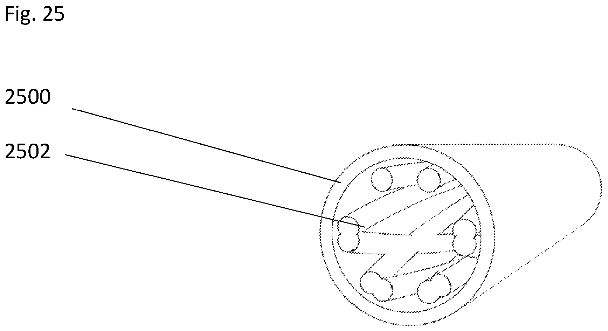

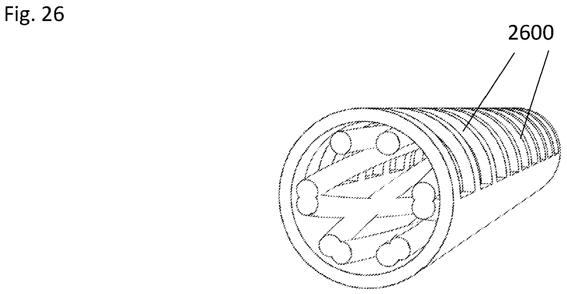

Reference is now made to Fig. 25, which illustrates the treatment object of any of Figs 21 to 23, covered with an insulating covering 2500, that insulates healthy tissues from the heating of the object. Referring to Fig. 26, the insulating covering may have windows 2600 built in, as will be shown and discussed hereinbelow, to allow for contact with the tumor tissue. The windows may be regularly spaced, or may be aligned with the tumors so that the spring-like outer structure is brought into contact with the tumors and shuttered away from healthy tissue. The insulating covering may be constructed per patient depending on the individual's location of tumors.

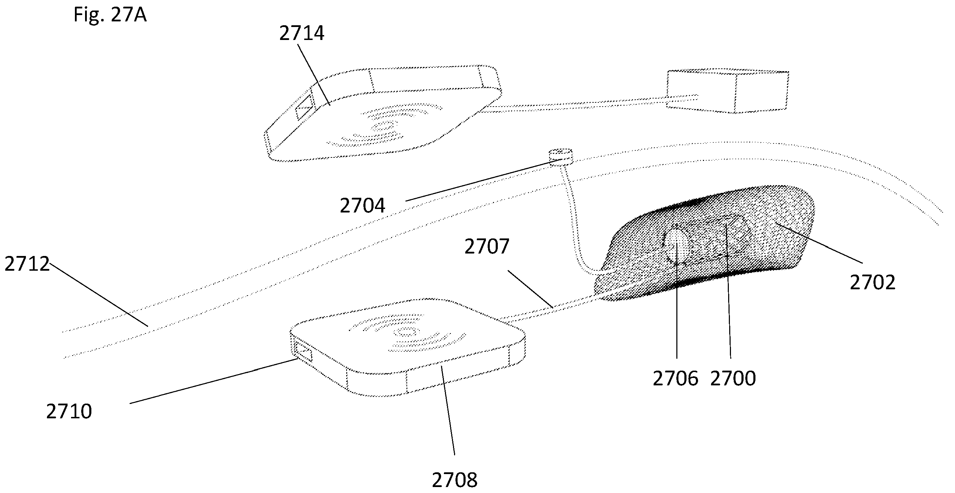

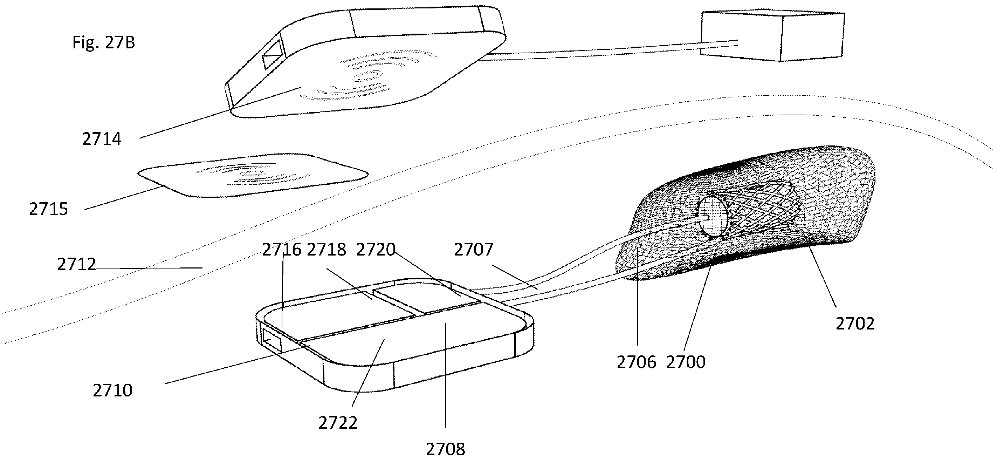

Reference is now made to Fig. 27A, which is a simplified drawing showing the treatment object 2700 inside an organ 2702 treating a tumor. The treatment object has a connection to a valve 2704 for insertion of chemicals for chemotherapy or the like. The bladder 2706 may be pumped up as needed, and a power connection 2707 supplies power for heating the spring like structure from antenna device 2708. The antenna device includes a microprocessor 2710 and may further include a battery. The antenna and treatment object are placed inside the body of the patient indicated by body wall 2712 and a transmitter 2714 located externally provides power for the treatment. It is noted in this embodiment that the valve 2704 is placed externally of the body so that it can be filled from outside. In other embodiments the antenna and treatment object may be fully contained within the body so as to reduce the chances of infection, as will be shown in greater detail hereinbelow. Reference is now made to Fig. 27B which shows the antenna device 2708 with lid 2715 removed. In an embodiment a container of gas or liquid 2716 is held inside the antenna device 2708 and is pumped using pump 2718 via pipe 2720 so that there is no breach of the body wall, thus lowering the chance of infection. A battery may be placed in compartment 2722. Again, chemicals for chemotherapy may be provided and the pump in 2718 may be switched between pumping chemicals and pumping fluid for filling the bladder.

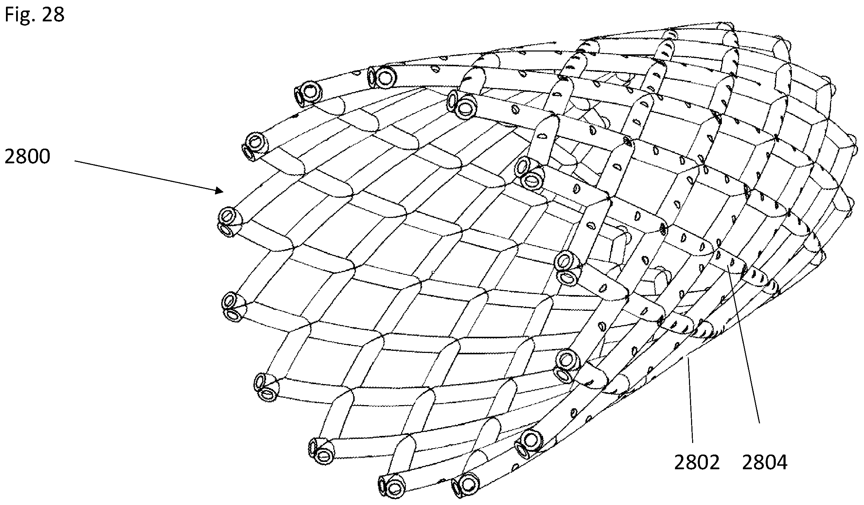

Fig. 28 illustrates a spring-like structure 2800 or wire mesh, which is made of hollow pipes 2802 having holes 2804. The pipes are used to pump chemotherapy agents and the holes are used to dripfeed the agents into the tumor. As discussed, the spring-like structure or wire mesh expands as required, and may provide the heating surface as well.

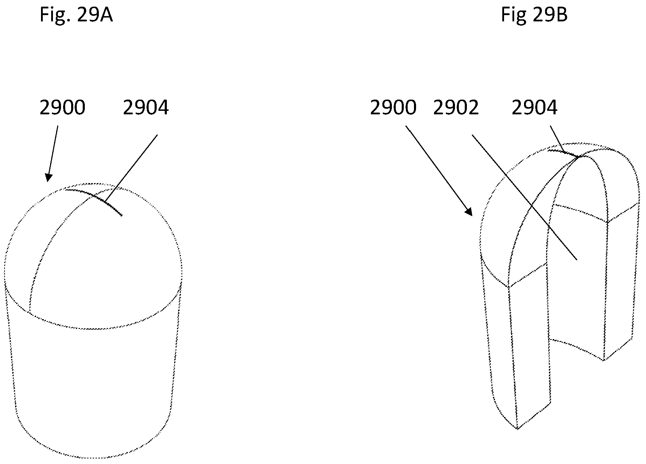

Figs 29A and 29B are perspective and section views of a bung-like device 2900 that is pressed by the pumping pressure into the holes in the pipes. The bung-like device has a hollow interior 2902 and a slit 2904. As the bung-like device is pressed into the hole the slit momentarily opens to release the pressure and drip some chemotherapy agent into the tumor.

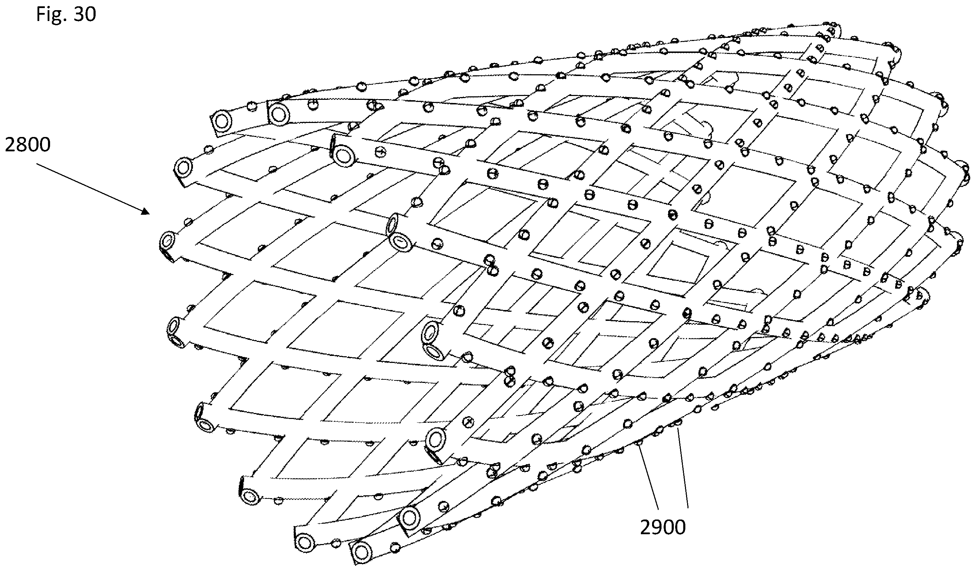

Reference is now made to Fig. 30, which illustrates the same springlike structure shown in Fig. 28, with the bung-like devices 2900 protruding from the holes. The bung-like devices not only drip feed chemotherapy agent into the tumor but also press into the walls of the tumor, increasing the surface area exposed to the treatment object.

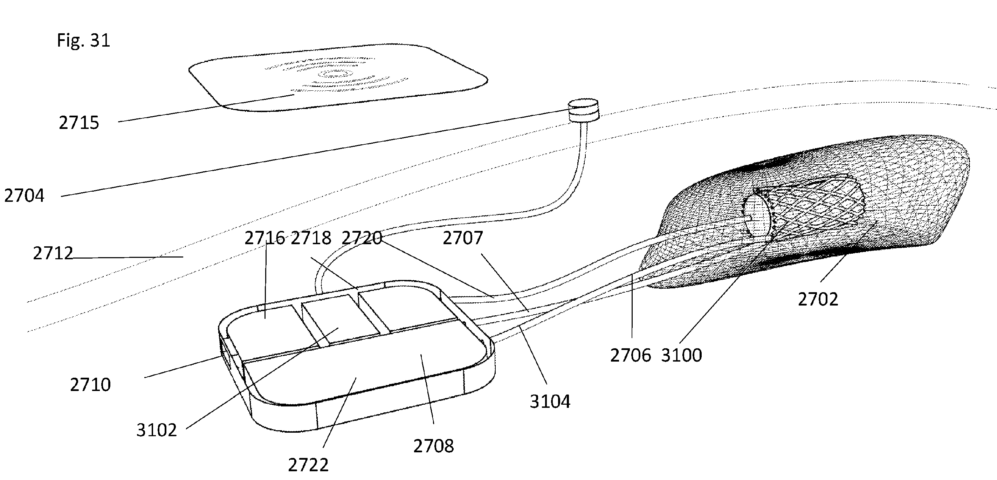

Reference is now made to Fig. 31, which is a simplified drawing showing a further embodiment in which treatment object 3100 is located inside an organ 2702 treating a tumor. The treatment object has a connection to a valve 2704 for insertion of chemicals to a tank 3102 for chemotherapy or the like. The tank 3102 is connected via pipe 3104 to the spring-like structure 3106. The bladder 2706 may be pumped up as needed, and a power connection 2707 supplies power for heating the spring like structure from antenna device 2708. The antenna device includes a microprocessor 2710 and may further include a battery. The antenna and treatment object are placed inside the body of the patient indicated by body wall 2712 and a transmitter 2714 located externally provides power for the treatment. It is noted in this embodiment that the valve 2704 is placed externally of the body so that it can be filled from outside. Lid 2715 removed to show the inside of antenna device 208. A container of gas or liquid 2716 is held inside the antenna device 2708 and is pumped using pump 2718 via pipe 2720 so that there is no breach of the body wall, thus lowering the chance of infection. Container 3102 contains chemotherapy agent as mentioned and the pump in 2718 may be switched between pumping chemicals from tank 3102 via pipe 3104, and pumping fluid for filling the bladder from container 2716 via pipe 2720. A battery may be placed in compartment 2722.

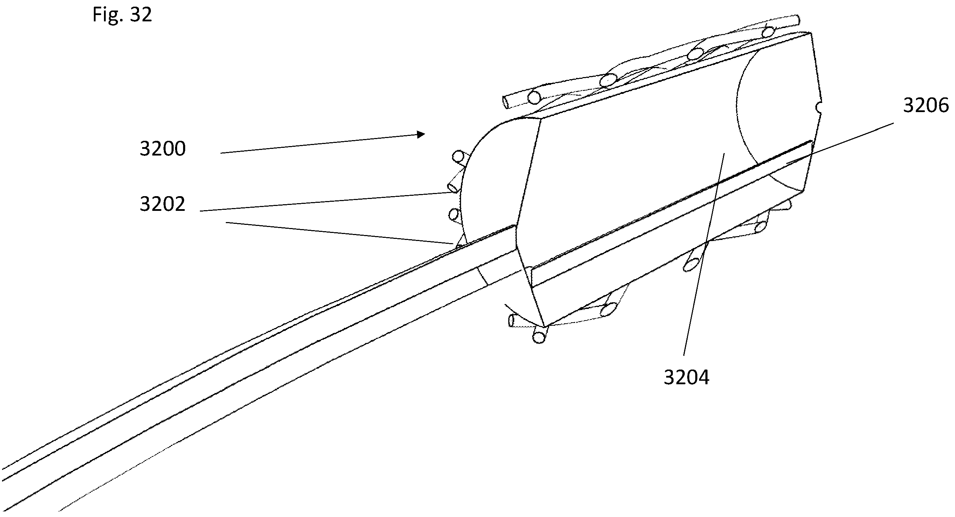

Reference is now made to Fig. 32, which is a cross-sectional view of treatment object 3200 comprising a spring-like member 3202 located over an expanded bladder 3204. A heating element 3206 is placed in the bladder and connected to elements of the spring-like member 3202. As an alternative the heating element may be built into the antenna device 2708 and hot fluid may be pumped via a pipe to heat the spring-like member.

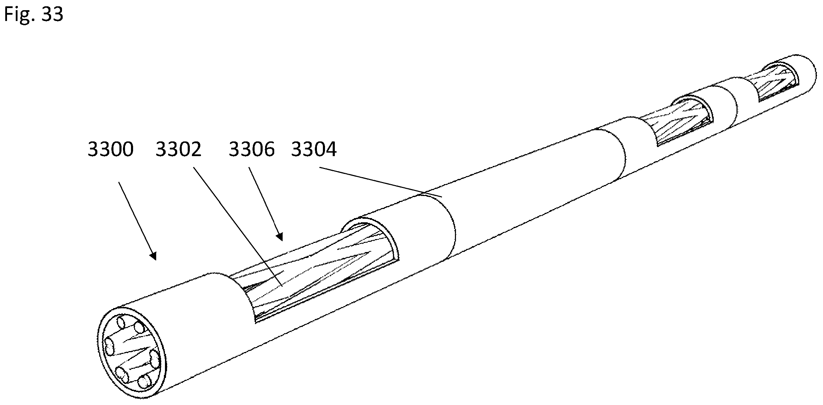

Reference is now made to Fig. 33, which illustrates a treatment object 3300 having a spring-like structure 3302 inside an insulating jacket 3304. The insulating jacket protects healthy tissue from the heat produced by the treatment body. However windows 3306 are custom made in the insulating jacket to expose the spring-like structure at intervals tailored so that when the treatment object is correctly positioned, all known tumors are located against one of the windows.

In general, tumor cells are fast growing cells and are more sensitive to heat than non-tumor cells and thus the device at its preferred temperature may effectively have a differential killing rate that kills tumor cells and leaves other cells unaffected.

In use, the treatment body 3300 is threaded to the treatment point in the same way that a stent is guided to the treatment point to align windows with tumors. In an embodiment, accurate placement may be guided on the basis of metallic markers placed in the tumors during a scan. During the scan, each tumor is marked with such a marker and then subsequently a device according to the embodiments is injected onto each marker.

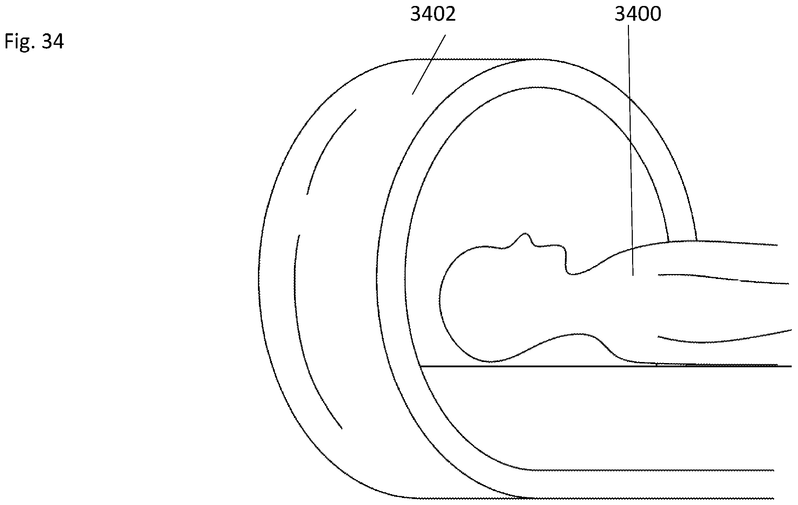

As explained, using a suitable antenna, the object may be heated inductively using an external field. This is particularly useful for multiple tumors, where separate objects may be injected into each tumor and then the patient may be placed in an electric field, say induced by a coil, and all the devices may be powered together. Reference is now made to Fig. 34, which illustrates patient 3400 placed in a device 3402 that may for example be similar to an MRI scanner. That is to say the devices has the coils of the MRI scanner but does not require the sensors. The patient is free to get up and walk around at intervals and then return to the coils to continue the treatment. Likewise, in a wired version in which the patient is connected via wires to an external power source, the patient may be enabled to disconnect the power source and walk around and then return and reconnect to continue the treatment. As a further embodiment, the power source may be mobile and connected to the patient. It is noted that not much power is required from the power source since the body is at or around 36.5 degrees and the preferred operating temperature is generally around 43 - 46 degrees, with some variation for the kind of tumor.

An embodiment is operated by pulsing the power, and this gives more accurate control of the temperature. This is because the pulse is for a clearly defined amount of time, thus providing a defined amount of energy at the given frequency and ensuring that the object is kept at the design temperature for a desired amount of time. General heating for an undefined amount of time may lead to excess heating of the object and cause injury to the patient. In addition to the pulsing that gives the defined amount of energy, the object may be heated for defined intervals and left for defined intervals so that the design temperature is not exceeded. For example the object may be heated for a minute to reach the design temperature. The heating process is stopped for say 30 seconds as the stored energy in the object dissipates to the tumor, and then the cycle is repeated to give a total exposure time of the tumor tissue to a specific energy in Joules. Given the heat capacity of the object, the relationship between the temperature and the energy can be determined so as to know how much energy is flowing into the tumor. The above-defined process may be continuous for an hour, and then may be repeated a day later as the tumor attempts to recover, which is a time at which the tumor is particularly vulnerable.

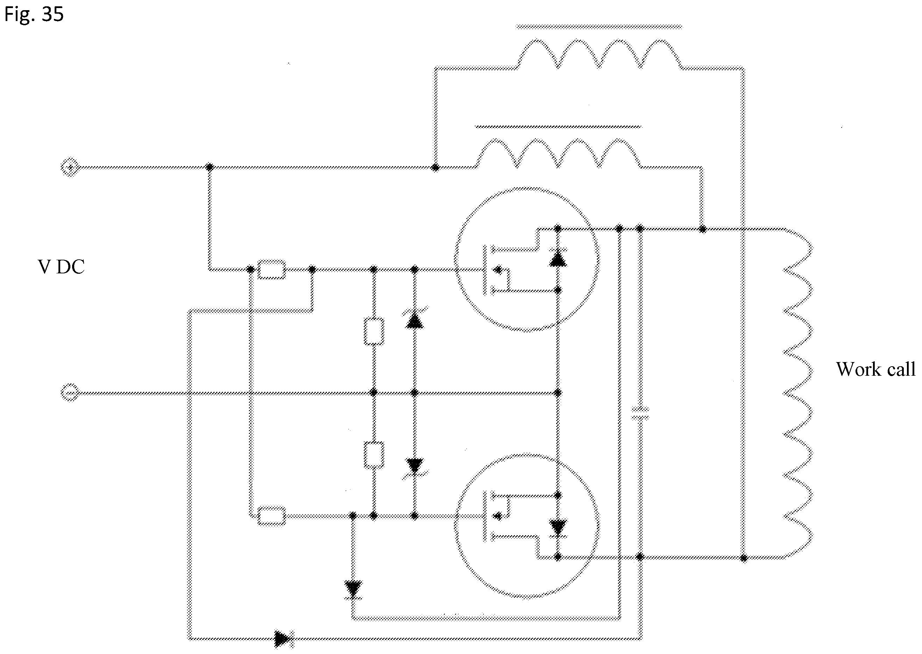

Reference is now made to Fig. 35, which is a simplified circuit diagram for a transmitter device for powering objects of the present embodiments. In the circuit diagram a transmission frequency is selected for maximum efficiency so that the objects may reach their design temperature within a minute. A transmission frequency of between 300 and 400 KHz using sine waves with high peak-to peak values, may be selected for objects that are around 0.4mm in diameter to maximize the skin effect. At around 1MHz there is very little skin effect. The transmitter may be cooled to be kept at a maximum efficiency temperature. A duty cycle of transmission pulses and intervals may be set up to maintain the objects at their design temperature. The duty cycle may vary due to the nature of the tumor or its position in the body, the amount of fatty tissue and the size and extent of local blood vessels, which all affect the flow of energy.

In the above embodiments, the object has been an expandable spring-like construction or even a wire net, placed over a bladder. The object may be delivered inside an outer envelope and released at or in proximity to the tumor, where the wire net then expands to a preset extent. The spring-like structure comprises an electrical resistor that heats under the influence of electrical current. As the tumor is destroyed layer by layer the balloon is expanded so that the wire net is in contact with the remainder of the tumor. As an alternative to wire the material may comprise a polymer with metallic particles. In order not to reduce concentration as the device expands, the material may be in folded layers, say of Milar sheet coated with metal which then unfolds.

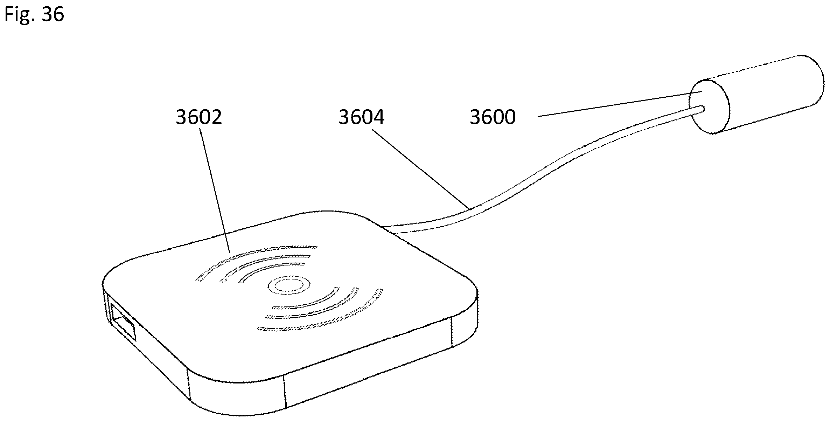

Referring now to Fig. 36, the spring-like structure is dispensed with. Instead a bladder 3600 is connected to an antenna device 3602 via a pipe 3604. The pipe 3604 supplies warmed liquid that both fills the bladder and heats the tumor.

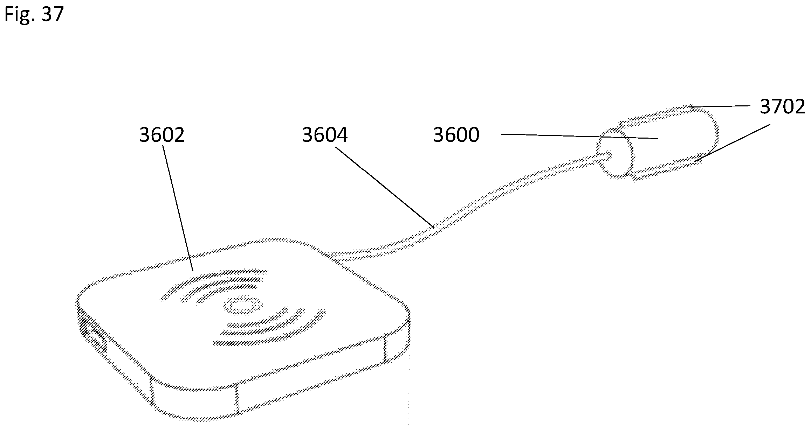

Reference is now made to Fig. 37, which shows a variation of the bladder of Fig. 36, in which flaps 3702 are provided on the sides of the bladder. The bladder may be stitched by the surgeon to any convenient attachment point to ensure that the bladder presses against the tumor.

The object according to some of the above embodiments, is particularly useful for destroying colon tumors which are today only treatable by surgical excision. Due to peristaltic action of the colon wall, the tumor cells have a tendency to be shed and to spread to other places. The object presses against the inner wall, preventing cells from breaking free, and also acts relatively fast in killing the exposed cells first, thus reducing the chances of the cancer spreading.

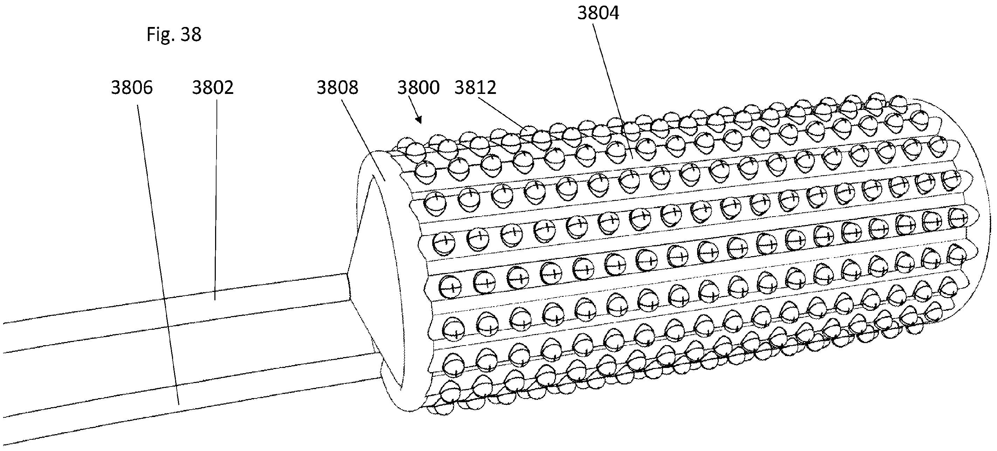

Reference is now made to Figs 38 - 40 which show a variation of the embodiments of Figs 36 and 37 to provide both heating and chemotherapy. An object 3800 comprises an inner bladder 3801 and an outer sheath 3804 with tubules 3810. The inner bladder 3801 is filled with heating fluid. The heating fluid may be heated in the antenna device or may be heated by a heating element in the bladder. The bladder fluid is provided via pipe 3802. Outer sheath 3804 is supplied via a separate pipe 3806 and includes a surrounding pipe 3808 and the tubules 3810. The tubules extend along the length of the object and have holes that are filled by the bung-like devices 3812 discussed hereinabove with slits, to provide drip-feeding. The outer sheath is filled with chemotherapy agent from pipe 3806 which is distributed to the tubules 3810 via surrounding pipe 3808 and is then drip fed into the tumor. It is noted that the bladder is shown as round, but in practice may be any shape, and in particular may be designed to fit the specific shape and size of a given tumor, in including flat. Thus the tumor may be imaged and a specific shape chosen as appropriate, and then made using suitable one-time manufacturing methods such as 3D printing.

As used herein, the singular form "a", "an" and "the" include plural references unless the context clearly dictates otherwise. For example, the term "a unit" or "at least one unit" may include a plurality of units, including combinations thereof.

The words "example" and "exemplary" are used herein to mean "serving as an example, instance or illustration". Any embodiment described as an "example or "exemplary" is not necessarily to be construed as preferred or advantageous over other embodiments and/or to exclude the incorporation of features from other embodiments.

Throughout this application, various examples of the present disclosure may be presented in a range format. It should be understood that the description in range format is merely for convenience and brevity and should not be construed as an inflexible limitation on the scope of the present invention which is defined by the appended claims.

Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Although the invention has been described in conjunction with specific embodiments thereof, it is evident that many alternatives, modifications and variations will be apparent to those skilled in the art.

The invention is not limited to the described examples and embodiments and is defined by the appended claims.

## Claims

1. A device for treatment of a tumor comprising:
a treatment object (12, 14, 401, 601, 701, 2200) configured for placement about the tumor, the treatment object comprising a heating surface configured to reach a treatment temperature, the treatment temperature being higher than a patient body temperature;
an energy source configured to provide energy to reach said treatment temperature, said energy source being located externally to said treatment object; and
an outwardly expandable spring (2202) around said treatment object, wherein the spring is configured to expand under temperature control;
**characterized in that** the device comprises a magnetic field source configured to move the treatment object in steps through a medium using magnetic pulses and to locate the treatment object at a desired location in the medium to treat said tumor.

2. The device of claim 1, wherein said energy source is a radio frequency transmitter configured to transmit energy at a predetermined frequency, and said treatment object comprises an antenna for wireless reception at said predetermined frequency.

3. The device of claim 1, wherein said externally located energy source comprises a battery, or wherein said externally located energy source comprises a laser, the laser being connected to said treatment object via an optical fiber.

4. The device of any one of the preceding claims, further comprising a needle for placement of said treatment object, wherein the treatment object is located within the needle, for accurate placement of said treatment object at a predetermined location.

5. The device of claim 1, further comprising a placement device for placement of said treatment object, wherein the treatment object is held for accurate placement of said treatment object at a predetermined location.

6. The device of claim 4, wherein the treatment object is attached to at least one wire, the wire remaining connected as the treatment object is expelled from said needle.

7. The device of any one of the preceding claims, further comprising a thermostat for regulating said treatment object at said treatment temperature.

8. The device of any one of the preceding claims, having a diameter larger than 0.05 mm and less than one millimeter.

9. The device of any one of the preceding claims, wherein said treatment temperature in degrees Celsius is one member of the group consisting of:
greater than 38 degrees;
greater than 39.5 degrees;
less than 55 degrees;
between 39.5 degrees and 55 degrees;
between 40 degrees and 50 degrees;
between 40 degrees and 48 degrees;
between 41 degrees and 47 degrees;
between 42 degrees and 46 degrees;
between 43 degrees and 45 degrees;
between 43 degrees and 44 degrees;
about 43.5 degrees; and
precisely 43.5 degrees.

10. The device of claim 1, comprising a bladder located inside said spring, said bladder being expandable under external control.

11. The device of claim 1, wherein said object comprises an expandable bladder, said heating surface being an external surface of said bladder.

## Patentansprüche

1. Vorrichtung zur Behandlung eines Tumors, umfassend:
ein Behandlungsobjekt (12, 14, 401, 601, 701, 2200), welches für eine Platzierung um den Tumor herum eingerichtet ist, wobei das Behandlungsobjekt eine Heizfläche umfasst, welche dazu eingerichtet ist, eine Behandlungstemperatur zu erreichen, wobei die Behandlungstemperatur höher ist als eine Patientenkörpertemperatur,
eine Energiequelle, welche dazu eingerichtet ist, Energie bereitzustellen, um die Behandlungstemperatur zu erreichen, wobei die Energiequelle außerhalb des Behandlungsobjekts angeordnet ist; und
eine nach außen ausdehnbare Feder (2202) um das Behandlungsobjekt herum, wobei die Feder dazu eingerichtet ist, sich unter einer Temperatursteuerung auszudehnen;
**dadurch gekennzeichnet, dass** die Vorrichtung eine Magnetfeldquelle umfasst, welche dazu eingerichtet ist, das Behandlungsobjekt unter Verwendung magnetischer Impulse in Stufen durch ein Medium zu bewegen und das Behandlungsobjekt an einer gewünschten Stelle in dem Medium zu lokalisieren, um den Tumor zu behandeln.

2. Vorrichtung nach Anspruch 1, wobei die Energiequelle ein Radiofrequenztransmitter ist, welcher dazu eingerichtet ist, Energie mit einer vorbestimmten Frequenz zu übertragen, und das Behandlungsobjekt eine Antenne zum drahtlosen Empfang auf der vorbestimmten Frequenz umfasst.

3. Vorrichtung nach Anspruch 1, wobei die extern angeordnete Energiequelle eine Batterie umfasst oder wobei die extern angeordnete Energiequelle einen Laser umfasst, wobei der Laser über eine optische Faser mit dem Behandlungsobjekt verbunden ist.

4. Vorrichtung nach einem der vorhergehenden Ansprüche, ferner umfassend eine Nadel zum Platzieren des Behandlungsobjekts, wobei das Behandlungsobjekt innerhalb der Nadel angeordnet ist, für eine akkurate Platzierung des Behandlungsobjekts an einer vorbestimmten Stelle.

5. Vorrichtung nach Anspruch 1, ferner umfassend eine Platzierungsvorrichtung zum Platzieren des Behandlungsobjekts, wobei das Behandlungsobjekt für eine akkurate Platzierung des Behandlungsobjekts an einer vorbestimmten Stelle gehalten wird.

6. Vorrichtung nach Anspruch 4, wobei das Behandlungsobjekt an wenigstens einem Draht angebracht ist, wobei der Draht verbunden bleibt, wenn das Behandlungsobjekt aus der Nadel ausgestoßen wird.

7. Vorrichtung nach einem der vorhergehenden Ansprüche, ferner umfassend ein Thermostat zum Regulieren des Behandlungsobjekts auf die Behandlungstemperatur.

8. Vorrichtung nach einem der vorhergehenden Ansprüche, welche einen Durchmesser aufweist, welcher größer als 0,05 mm und kleiner als ein Millimeter ist.

9. Vorrichtung nach einem der vorhergehenden Ansprüche, wobei die Behandlungstemperatur in Grad Celsius ein Element der Gruppe ist, bestehend aus:
größer als 38 Grad;
größer als 39,5 Grad;
niedriger als 55 Grad;
zwischen 39,5 Grad und 55 Grad;
zwischen 40 Grad und 50 Grad;
zwischen 40 Grad und 48 Grad;
zwischen 41 Grad und 47 Grad;
zwischen 42 Grad und 46 Grad;
zwischen 43 Grad und 45 Grad;
zwischen 43 Grad und 44 Grad;
etwa 43,5 Grad; und
genau 43,5 Grad.

10. Vorrichtung nach Anspruch 1, umfassend eine Blase, welche innerhalb der Feder angeordnet ist, wobei die Blase unter einer externen Steuerung ausdehnbar ist.

11. Vorrichtung nach Anspruch 1, wobei das Objekt eine ausdehnbare Blase umfasst, wobei die Heizfläche eine äußere Fläche der Blase ist.

## Revendications

1. Dispositif pour le traitement d'une tumeur comprenant :
un objet de traitement (12, 14, 401, 601, 701, 2200) configuré pour être placé aux environs de la tumeur, l'objet de traitement comprenant une surface chauffante configurée pour atteindre une température de traitement, la température de traitement étant supérieure à une température corporelle du patient ;
une source d'énergie configurée pour fournir de l'énergie pour atteindre ladite température de traitement, ladite source d'énergie étant positionnée à l'extérieur dudit objet de traitement ; et
un ressort (2202) extensible vers l'extérieur autour dudit objet de traitement, dans lequel le ressort est configuré pour s'étendre sous une régulation de température ;
**caractérisé en ce que** le dispositif comprend une source de champ magnétique configurée pour déplacer l'objet de traitement par étapes à travers un milieu à l'aide d'impulsions magnétiques et pour positionner l'objet de traitement à un emplacement souhaité dans le milieu pour traiter ladite tumeur.

2. Dispositif selon la revendication 1, dans lequel ladite source d'énergie est un émetteur radiofréquence configuré pour émettre de l'énergie à une fréquence prédéterminée, et ledit objet de traitement comprend une antenne pour une réception sans fil à ladite fréquence prédéterminée.

3. Dispositif selon la revendication 1, dans lequel ladite source d'énergie positionnée à l'extérieur comprend une batterie, ou dans lequel ladite source d'énergie positionnée à l'extérieur comprend un laser, le laser étant relié audit objet de traitement via une fibre optique.

4. Dispositif selon l'une quelconque des revendications précédentes, comprenant en outre une aiguille pour un placement dudit objet de traitement, dans lequel l'objet de traitement est positionné à l'intérieur de l'aiguille, pour un placement précis dudit objet de traitement à un emplacement prédéterminé.

5. Dispositif selon la revendication 1, comprenant en outre un dispositif de placement pour un placement dudit objet de traitement, dans lequel l'objet de traitement est maintenu pour un placement précis dudit objet de traitement à un emplacement prédéterminé.

6. Dispositif selon la revendication 4, dans lequel l'objet de traitement est fixé à au moins un fil, le fil restant relié lorsque l'objet de traitement est expulsé de ladite aiguille.

7. Dispositif selon l'une quelconque des revendications précédentes, comprenant en outre un thermostat pour la régulation dudit objet de traitement à ladite température de traitement.

8. Dispositif selon l'une quelconque des revendications précédentes, ayant un diamètre supérieur à 0,05 mm et inférieur à un millimètre.

9. Dispositif selon l'une quelconque des revendications précédentes, dans lequel ladite température de traitement en degrés Celsius est un élément du groupe consistant en :
plus de 38 degrés ;
plus de 39,5 degrés ;
moins de 55 degrés ;
entre 39,5 degrés et 55 degrés ;
entre 40 degrés et 50 degrés ;
entre 40 degrés et 48 degrés ;
entre 41 degrés et 47 degrés ;
entre 42 degrés et 46 degrés ;
entre 43 degrés et 45 degrés ;
entre 43 degrés et 44 degrés ;
environ 43,5 degrés ; et
exactement 43,5 degrés.

10. Dispositif selon la revendication 1, comprenant une vessie positionnée à l'intérieur dudit ressort, ladite vessie étant extensible sous une commande externe.

11. Dispositif selon la revendication 1, dans lequel ledit objet comprend une vessie extensible, ladite surface chauffante étant une surface externe de ladite vessie.
